# EUROPEAN PATENT APPLICATION

(11) **EP 4 445 891 A1**
(43) Date of publication of application: **16.10.2024**
(21) Application number: 22904346.8
(22) Date of filing: 09.12.2022
(51) Int. Cl.: A61K 6/60, A61K 6/30, A61K 6/54, A61K 6/62, A61K 6/71, A61K 6/887

(54) **DENTAL CURABLE COMPOSITION**

(30) Priority: 09.12.2021 JP 2021200444
(71) Applicant: Kuraray Noritake Dental Inc., Kurashiki-shi, Okayama 710-0801 (JP)
(72) Inventor: ATAKA, Kensuke, Tainai-shi, Niigata 959-2653 (JP); NOJIRI, Yamato, Tainai-shi, Niigata 959-2653 (JP)
(74) Representative: D Young & Co LLP
(86) International application number: PCT/JP2022/045570
(87) International publication number: WO 2023/106419

(57) **Abstract**

The present invention provides a dental curable composition that exhibits excellent mechanical strength in its cured product, and possesses high paste flowability and sufficient formability, previously deemed conflicting paste properties. The present invention relates to a dental curable composition comprising a polymerizable monomer (A), a filler (B), and a polymerization initiator (C), wherein:
the polymerizable monomer (A) comprises 0 parts or more by mass and less than 15 parts by mass of a hydrophilic polymerizable monomer (A-1a) having no acidic group, and 55 parts or more by mass of a hydrophobic polymerizable monomer (A-1b) having no acidic group in total 100 parts by mass of the polymerizable monomer (A), and the hydrophilic polymerizable monomer (A-1a) having no acidic group has a solubility of 4.5 g/L or more in water at 25°C ,
the filler (B) comprises 1 to 60 parts by mass of a spherical filler (B-1) having an average particle diameter of 0.01 to 1 µm, 40 to 99 parts by mass of an irregularly shaped filler (B-2) having an average particle diameter of 0.5 to 6.0 µm, and 0 to 10 parts by mass of an irregularly shaped fine particulate filler (B-3) having an average particle diameter of less than 0.5 µm in total 100 parts by mass of the filler (B), and the spherical filler (B-1) is hydrophobized with a hydrophobizing agent (x-1), and
the polymerization initiator (C) comprises a photopolymerization initiator (C-1).

## Description

### TECHNICAL FIELD

The present invention relates to dental curable compositions.

### BACKGROUND ART

Dental curable compositions comprise a mixture of polymerizable monomer, filler, and polymerization initiator, and are currently the most commonly employed materials for the filling and restoration of tooth defects or cavities. These compositions are categorized into a number of types based on their intended uses. Distinct properties are necessary for each application. For example, dental curable compositions intended for dental cements require high flowability. In particular, dental curable compositions employed as dental abutment construction materials demand high formability, or form retention, as well as high mechanical strength. The desired properties for each application can be achieved by incorporating specific components into the compositions, or by adjusting variables such as the ratios of components.

For example, Patent Literature 1 discloses a dental curable composition that combines spherical fillers and irregularly shaped fillers to concurrently attain superior properties relevant to science and engineering, such as paste flowability and the mechanical strength of the cured product, which have been difficult to achieve in the past.

The dental curable composition described in Patent Literature 2 uses a filler treated with a surfactant and a hydrophobizing agent, allowing for enhanced formability while maintaining superior science and engineering-related properties such as paste flowability and the mechanical strength of the cured product, without using a fine particulate filler with a particle size below 0.05 µm, a commonly used thixotropic modifier.

### CITATION LIST

### Patent Literature

Patent Literature 1: JP 2005-170813 A
Patent Literature 2: JP 2020-040937 A

### SUMMARY OF INVENTION

### Technical Problem

In the dental curable composition described in Patent Literature 1, it is stated that the inclusion of fillers with an average particle diameter of less than 0.05 µm is optional, and that essentially only two types of fillers can be used to achieve good paste flowability and favorable mechanical strength in the cured product.

However, Patent Literature 1 does not address the formability of the paste during build-up for abutment construction, and merely explains that a filler with a particle diameter of less than 0.05 µm can be used as a thixotropic modifier.

In abutment construction, shaping the built-up paste to match the shape of the abutment tooth is essential, emphasizing the importance of paste formability. However, there is no direct mentioning of this in Patent Literature 1.

Examinations by the present inventors revealed that the dental curable composition described in Patent Literature 1 exhibits impaired flowability when a filler having an average particle diameter of less than 0.05 µm is incorporated as a thixotropic modifier to prepare a paste that possesses formability, an important quality as a dental abutment construction material.

According to the assessment conducted by the present inventors, the dental curable composition described in Patent Literature 2 can enhance the paste formability to some extent with the use of fillers treated with a surfactant and a hydrophobizing agent, without using a thixotropic modifier. However, this improved formability is not sufficient for serving as a dental abutment construction material, and attempts to enhance formability by increasing the filler content result in a significant reduction in paste flowability. That is, it has been determined that the dental curable composition of Patent Literature 2 cannot satisfy a high level of formability sufficient for dental abutment construction materials while achieving high paste flowability.

Applications that require paste flowability, such as dental cements, can be managed by changing the formulation of the dental curable composition to achieve the desired flowability. Similarly, applications requiring paste formability, such as dental abutment construction materials, can be managed by changing the formulation of the dental curable composition to attain the desired formability. Consequently, the challenge of providing a dental curable composition possessing the two conflicting properties-paste flowability and paste formability-has seldom been explored due to its complexity. Instead, it has been approached for each application by changing formulations.

It is accordingly an object of the present invention to provide a dental curable composition having outstanding mechanical strength in its cured product, along with paste properties involving high flowability and sufficient formability.

### Solution to Problem

The present inventors conducted continual intensive studies to find a solution to the foregoing issues, and found that combining spherical fillers and irregularly shaped fillers in specific proportions, along with maintaining the hydrophilic polymerizable monomer content below a certain level within polymerizable monomers, results in a paste that exhibits high formability when at rest and high flowability under external force. Through this finding, the present inventor has successfully provided a dental curable composition possessing both high paste flowability and sufficient formability, previously deemed conflicting paste properties. Building upon this finding, the present inventors conducted additional examinations, ultimately resulting in the completion of the present invention.

Specifically, the present invention includes the following.
[1] A dental curable composition comprising a polymerizable monomer (A), a filler (B), and a polymerization initiator (C), wherein:
   the polymerizable monomer (A) comprises 0 parts or more by mass and less than 15 parts by mass of a hydrophilic polymerizable monomer (A-1a) having no acidic group, and 55 parts or more by mass of a hydrophobic polymerizable monomer (A-1b) having no acidic group in total 100 parts by mass of the polymerizable monomer (A), and the hydrophilic polymerizable monomer (A-1a) having no acidic group has a solubility of 4.5 g/L or more in water at 25°C. ,
   the filler (B) comprises 1 to 60 parts by mass of a spherical filler (B-1) having an average particle diameter of 0.01 to 1 µm, 40 to 99 parts by mass of an irregularly shaped filler (B-2) having an average particle diameter of 0.5 to 6.0 µm, and 0 to 10 parts by mass of an irregularly shaped fine particulate filler (B-3) having an average particle diameter of less than 0.5 µm in total 100 parts by mass of the filler (B), and the spherical filler (B-1) is hydrophobized with a hydrophobizing agent (x-1), and
   the polymerization initiator (C) comprises a photopolymerization initiator (C-1).
[2] The dental curable composition according to [1], wherein the irregularly shaped fine particulate filler (B-3) has an average particle diameter of less than 0.05 µm.
[3] The dental curable composition according to [1] or [2], wherein the filler (B) comprises 10 to 30 parts by mass of the spherical filler (B-1), 69 to 89 parts by mass of the irregularly shaped filler (B-2), and 1 to 5 parts by mass of the irregularly shaped fine particulate filler (B-3) in total 100 parts by mass of the filler (B).
[4] The dental curable composition according to any one of [1] to [3], wherein the hydrophobizing agent (x-1) comprises at least one selected from the group consisting of a silane coupling agent, a titanate-based coupling agent, an aluminate-based coupling agent, and a zirco-aluminate-based coupling agent.
[5] The dental curable composition according to any one of [1] to [4], wherein the irregularly shaped filler (B-2) is hydrophobized with a hydrophobizing agent (x-2).
[6] The dental curable composition according to [5], wherein the hydrophobizing agent (x-2) is a silane coupling agent having a polymerizable group and represented by the following general formula (1),

   Y-SiRₙX₍₃₋ₙ₎ (1),

   wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, n represents an integer of 0, 1, or 2, and R may be the same or different from each other when R is plural, and X may be the same or different from each other when X is plural.
[7] The dental curable composition according to [6], wherein Y is a group with a (meth)acryloyl group and an organic group bound to each other via a divalent group containing an oxygen atom, and the organic group bound to the (meth)acryloyl group via a divalent group containing an oxygen atom is an alkyl group having 5 to 11 carbon atoms.
[8] The dental curable composition according to any one of [1] to [7], wherein the polymerizable monomer (A) further comprises a polymerizable monomer (A-2) having an acidic group.
[9] The dental curable composition according to any one of [1] to [8], wherein the polymerization initiator (C) further comprises a chemical polymerization initiator (C-2).
[10] The dental curable composition according to [9], which comprises a first agent and a second agent,
   the first agent comprising the polymerizable monomer (A), the filler (B), and a chemical polymerization accelerator (D-2),
   the second agent comprising the polymerizable monomer (A), the filler (B), and the chemical polymerization initiator (C-2).
[11] The dental curable composition according to [10], wherein the chemical polymerization accelerator (D-2) comprises a thiourea compound and/or a salt of an aromatic sulfinic acid.
[12] A dental abutment construction material comprising a dental curable composition of any one of [1] to [11].
[13] A dental resin cement comprising a dental curable composition of any one of [1] to [11].

### Advantageous Effects of Invention

According to the present invention, a dental curable composition can be provided that has outstanding mechanical strength in its cured product, along with paste properties involving high flowability and sufficient formability.

The present invention satisfies two conflicting properties-high flowability and high formability-by the altering properties of a dental curable composition paste that is highly formable when at rest and highly flowable under external force (for example, contact pressure). This enables a dental curable composition of a single formulation to be used in different applications, including dental cements, which demand flowability, and dental abutment construction materials, which necessitate formability, without the need for changing formulations.

### BRIEF DESCRIPTION OF DRAWINGS

[FIG. 1] A photograph representing the shape of a paste with a formability rating of 5 at the time of weight measurement.
[FIG. 2] A photograph representing the shape of a paste with a formability rating of 5 after 10 minutes at 37°C from weight measurement.
[FIG. 3] A photograph representing the shape of a paste with a formability rating of 1 at the time of weight measurement.
[FIG. 4] A photograph representing the shape of a paste with a formability rating of 1 after 10 minutes at 37°C from weight measurement.

### DESCRIPTION OF EMBODIMENTS

A dental curable composition of the present invention comprises a polymerizable monomer (A), a filler (B), and a polymerization initiator (C), wherein:
the polymerizable monomer (A) comprises 0 parts or more by mass and less than 15 parts by mass of a hydrophilic polymerizable monomer (A-1a) having no acidic group, and 55 parts or more by mass of a hydrophobic polymerizable monomer (A-1b) having no acidic group in total 100 parts by mass of the polymerizable monomer (A), and the hydrophilic polymerizable monomer (A-1a) having no acidic group has a solubility of 4.5 g/L or more in water at 25°C,
the filler (B) comprises 1 to 60 parts by mass of a spherical filler (B-1) having an average particle diameter of 0.01 to 1 µm, 40 to 99 parts by mass of an irregularly shaped filler (B-2) having an average particle diameter of 0.5 to 6.0 µm, and 0 to 10 parts by mass of an irregularly shaped fine particulate filler (B-3) having an average particle diameter of less than 0.5 µm in total 100 parts by mass of the filler (B), and the spherical filler (B-1) is hydrophobized with a hydrophobizing agent (x-1), and
the polymerization initiator (C) comprises a photopolymerization initiator (C-1).

While the reasons why the configuration of the present invention can produce the outstanding effects described above remain somewhat unclear, the following speculation has been made.

Generally, the hydrophilic interaction between polymerizable monomers and fillers is recognized as a factor contributing to increased paste viscosity. To reduce this increase in paste viscosity and diminish such hydrophilic interaction, it is contemplated to employ a polymerizable monomer with relatively low hydrophilicity and a filler with surface hydrophobic treatment. The interaction between the polymerizable monomer with relatively low hydrophilicity and the filler with surface hydrophobic treatment is notably weak. While this prevents a viscosity increase in the paste, providing flowability, it results in a composition with poor formability.

In a composition containing a polymerizable monomer of relatively low hydrophilicity and a filler with surface hydrophobic treatment, increasing the filler content enhances the contact area between the polymerizable monomer and the filler. This increase in contact area allows for sufficient formability even when the interactions between the polymerizable monomer and the filler are weak. These interactions are weaker than the hydrophilic interactions, and can be readily overcome with small force.

However, in such a paste with an increased ratio of filler content, the paste flowability is compromised when, for example, irregularly shaped fillers are used in increased amounts because these fillers entangle and hinder each other when force is applied to the paste, and increase the paste viscosity.

On the contrary, a dental curable composition of the present invention prevents compromising paste flowability under external force while possessing sufficient formability. This is achieved by incorporating a certain quantity of spherical fillers alongside other fillers, allowing the spherical fillers to produce a sliding effect under external force, and mitigating interference between the fillers.

This probably explains the altering properties of a dental curable composition of the present invention exhibiting high formability when at rest and high paste flowability under external force

### [Polymerizable Monomer (A)]

Radical polymerizable monomers are suitably employed as the polymerizable monomer (A) used in a dental curable composition of the present invention.

Examples of radical polymerizable monomers as polymerizable monomers (A) include (meth)acrylate polymerizable monomers, (meth)acrylamide polymerizable monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, mono-N-vinyl derivatives, and styrene derivatives. Preferred in view of curability are (meth)acrylate polymerizable monomers, and (meth)acrylamide polymerizable monomers.

The polymerizable monomer (A) can be broadly classified into polymerizable monomer (A-1) having no acidic group, and polymerizable monomer (A-2) having an acidic group.

In this specification, "(meth)acryl" is a collective term for methacryl and acryl.

### <Polymerizable Monomer (A-1) Having no Acidic Group>

In the present invention, the polymerizable monomer (A-1) having no acidic group can be divided into a hydrophilic polymerizable monomer (A-1a) having no acidic group with a solubility of 4.5 g/L or more in water at 25°C ; and a hydrophobic polymerizable monomer (A-1b) having no acidic group with a solubility of less than 4.5 g/L in water at 25°C.

### · Hydrophilic Polymerizable Monomer (A-1a) Having no Acidic Group

In a dental curable composition of the present invention, the polymerizable monomer (A) comprises less than 15 parts by mass of hydrophilic polymerizable monomer (A-1a) having no acidic group (hereinafter, also referred to simply as "hydrophilic polymerizable monomer (A-1a)") in total 100 parts by mass of polymerizable monomer (A).

Here, hydrophilic polymerizable monomer (A-1a) means a monomer that does not have an acidic group, and that has a solubility of 4.5 g/L or more in water at 25°C .

In a dental curable composition of the present invention, in view of satisfying both paste flowability and formability, the content of hydrophilic polymerizable monomer (A-1a) specified by its solubility in water is less than 15 parts by mass in total 100 parts by mass of polymerizable monomer (A).

The paste flowability decreases, and it is not possible to satisfy both flowability and formability when the content of hydrophilic polymerizable monomer (A-1a) in a dental curable composition of the present invention is 15 parts or more by mass in total 100 parts by mass of polymerizable monomer (A). It is therefore preferable that the content of hydrophilic polymerizable monomer (A-1a) in a dental curable composition of the present invention be 10 parts or less by mass, more preferably 5 parts or less by mass in total 100 parts by mass of polymerizable monomer (A). The content of hydrophilic polymerizable monomer (A-1a) may be 0 parts by mass in total 100 parts by mass of polymerizable monomer (A).

The hydrophilic polymerizable monomer (A-1a) enhances the wettability of the dental curable composition to tooth structures.

The hydrophilic polymerizable monomer (A-1a) is preferably a radical polymerizable monomer having no acidic group but having a polymerizable group. In view of ease of radical polymerization, the polymerizable group is preferably a (meth)acryloyloxy group and/or a (meth)acrylamide group.

Examples of the hydrophilic polymerizable monomer (A-1a) include:
hydrophilic (meth)acrylate monofunctional polymerizable monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and tetrahydrofurfuryl (meth)acrylate;
hydrophilic (meth)acrylate polyfunctional polymerizable monomers such as triethylene glycol di(meth)acrylate, tetraethylene glycol di(meth)acrylate, nonaethylene glycol di(meth)acrylate, 1,3-bis(methacryloyloxy)-2-propanol, and 1,2-bis(3-methacryloyloxy-2-hydroxypropoxy)ethane; and
hydrophilic (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, N,N-diethylacrylamide, and N-methacryloyloxyethylacrylamide.

In view of curability, preferred among these hydrophilic polymerizable monomers (A-1a) are (meth)acrylate polyfunctional polymerizable monomers, more preferably triethylene glycol dimethacrylate (commonly known as 3G), 1,3-bis(methacryloyloxy)-2-propanol, and 1,2-bis(3-methacryloyloxy-2-hydroxypropoxy)ethane (commonly known as #801), even more preferably 3G and #801.

The hydrophilic polymerizable monomer (A-1a) may be used alone, or two or more thereof may be used in combination.

### · Hydrophobic Polymerizable Monomer (A-1b) Having no Acidic Group

In a dental curable composition of the present invention, the polymerizable monomer (A) comprises a hydrophobic polymerizable monomer (A-1b) having no acidic group (hereinafter, also referred to simply as "hydrophobic polymerizable monomer (A-1b)").

Here, hydrophobic polymerizable monomer (A-1b) means a monomer that does not have an acidic group, and that has a solubility of less than 4.5 g/L in water at 25°C .

The use of hydrophobic polymerizable monomer (A-1b) diminishes the hydrophilic interaction between the polymerizable monomer and the filler when used with the filler (B) comprising a hydrophobically treated spherical filler (B-1), allowing for enhanced paste flowability.

The hydrophobic polymerizable monomer (A-1b) can also mitigate the stringiness (stringing tendency) of the dental curable composition, enhancing the mechanical strength and other properties of the cured product.

The hydrophobic polymerizable monomer (A-1b) is preferably a radical polymerizable monomer having no acidic group but having a polymerizable group. In view of ease of radical polymerization, the polymerizable group is preferably a (meth)acryloyloxy group and/or a (meth)acrylamide group.

Aside from hydrophobic monofunctional polymerizable monomers, other examples of the hydrophobic polymerizable monomer (A-1b) include crosslinkable polymerizable monomers such as aromatic bifunctional polymerizable monomers of hydrophobic nature, aliphatic bifunctional polymerizable monomers of hydrophobic nature, and tri- and higher-functional polymerizable monomers of hydrophobic nature.

Examples of the hydrophobic monofunctional polymerizable monomers include methyl (meth)acrylate, ethyl (meth)acrylate, butyl (meth)acrylate, benzyl (meth)acrylate, isobornyl (meth)acrylate, p-cumyl-phenoxyethylene glycol (meth)acrylate, 2-phenoxybenzyl (meth)acrylate, stearyl (meth)acrylate, dicyclopentanyl (meth)acrylate, butoxydiethylene glycol (meth)acrylate, and methoxypolyethylene glycol (meth)acrylate. Preferred among these are benzyl methacrylate, isobornyl methacrylate, and p-cumyl-phenoxyethylene glycol methacrylate.

Examples of the aromatic bifunctional polymerizable monomers of hydrophobic nature include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-methacryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-methacryloyloxydiethoxyphenyl)propane, 2,2-bis(4-methacηloyloxytriethoxyphenyl)propane, 2,2-bis(4-methacryloyloxytetraethoxyphenyl)propane, and 2,2-bis(4-methacryloyloxypentaethoxyphenyl)propane. More preferred are Bis-GMA and D-2.6E.

Examples of the aliphatic bifunctional polymerizable monomers include monoethylene glycol di(meth)acrylate, diethylene glycol di(meth)acrylate, propylene glycol di(meth)acrylate, butylene glycol di(meth)acrylate, neopentyl glycol di(meth)acrylate, 1,5-pentanediol di(meth)acrylate, 1,6-hexanediol di(meth)acrylate, 1,10-decanediol di(meth)acrylate, and 2,2,4-trimethylhexamethylenebis(2-carbamoyloxyethyl)di(meth)acrylate. Preferred among these are neopentyl glycol dimethacrylate (commonly known as NPG), 2,2,4-trimethylhexamethylenebis(2-carbamoyloxyethyl)dimethacrylate (commonly known as UDMA), and 1,10-decanediol dimethacrylate (commonly known as DD). More preferred are NPG, UDMA, and DD.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acr ylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacryl ate.

In view of ease of handling and the mechanical strength of the cured product, preferred among these hydrophobic polymerizable monomers (A-1b) are aromatic bifunctional polymerizable monomers, and aliphatic bifunctional polymerizable monomers.

The hydrophobic polymerizable monomer (A-1b) may be incorporated alone, or two or more thereof may be used in combination. An excessively high content of hydrophobic polymerizable monomer (A-1b) in a dental curable composition of the present invention may lead to a reduction in the wettability of the dental curable composition to tooth structures, and a subsequent decrease in adhesion. When the content is too low, the strong hydrophilic interaction between the polymerizable monomer and filler may result in insufficient paste flowability, possibly leading to a cured product with insufficient mechanical strength. The content of hydrophobic polymerizable monomer (A-1b) in a dental curable composition of the present invention is therefore 55 parts or more by mass, preferably 55 parts or more by mass, more preferably 70 parts or more by mass, even more preferably 85 parts or more by mass, particularly preferably 90 parts or more by mass, and may be 100 parts by mass in total 100 parts by mass of polymerizable monomer (A).

A certain preferred embodiment is, for example, a dental curable composition in which the content of hydrophobic polymerizable monomer (A-1b) is 55 parts or more by mass and 100 parts or less by mass in total 100 parts by mass of polymerizable monomer (A).

Another certain preferred embodiment, for example when a dental curable composition of the present invention comprises a hydrophilic polymerizable monomer (A-1a), is, for example, a dental curable composition in which the content of hydrophilic polymerizable monomer (A-1a) is more than 0 parts by mass and less than 15 parts by mass, and the content of hydrophobic polymerizable monomer (A-1b) is 85 parts or more by mass and less than 100 parts by mass in total 100 parts by mass of polymerizable monomer (A).

Yet another certain preferred embodiment, for example when a dental curable composition of the present invention comprises a polymerizable monomer (A-2) having an acidic group, is, for example, a dental curable composition in which the content of polymerizable monomer (A-2) having an acidic group is more than 0 parts by mass and 30 parts or less by mass, and the content of hydrophobic polymerizable monomer (A-1b) is 55 parts or more by mass and less than 100 parts by mass in total 100 parts by mass of polymerizable monomer (A).

In any of the embodiments above, the content of each monomer can be varied as appropriate based on the descriptions provided in this specification.

### <Polymerizable Monomer (A-2) Having an Acidic Group>

A dental curable composition of the present invention may comprise a polymerizable monomer (A-2) having an acidic group.

The polymerizable monomer (A-2) having an acidic group used in the present invention is preferably a radical polymerizable monomer having at least one acidic group and at least one polymerizable group. In view of adhesive properties, the acidic group present in the polymerizable monomer (A-2) having an acidic group is preferably an acidic group such as a phosphoric acid group, a pyrophosphoric acid group, a thiophosphoric acid group, a phosphonic acid group, a carboxylic acid group, or a sulfonic acid group.

In view of ease of radical polymerization, the polymerizable group present in the polymerizable monomer (A-2) having an acidic group is preferably a (meth)acryloyloxy group and/or a (meth)acrylamide group.

Examples of polymerizable monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl)hydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and acid chlorides, alkali metal salts, and amine salts of these.

Examples of polymerizable monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of polymerizable monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of polymerizable monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, and ammonium salts of these.

Examples of polymerizable monomers having a carboxylic acid group include monofunctional (meth)acrylic acid esters having one carboxyl group or an acid anhydride group thereof within the molecule, monofunctional (meth)acrylic acid esters having multiple carboxyl groups or acid anhydride groups thereof within the molecule, and acid chlorides, alkali metal salts, and ammonium salts of these.

Examples of the monofunctional polymerizable monomers having one carboxyl group or an acid anhydride group thereof within the molecule include (meth)acrylic acid, N-(meth)acryloyl glycine, N-(meth)acryloyl aspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyl tyrosine, N-(meth)acryloyl tyrosine, N-(meth)acryloyl phenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of the aforementioned compounds into an acid anhydride group

Examples of the monofunctional polymerizable monomers having multiple carboxyl groups or acid anhydride groups thereof within the molecule include 6-(meth)acryloyloxyhexane-1,1 -dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of polymerizable monomers having a sulfonic acid group include 2-sulfoethyl (meth)acrylate, and acid chlorides, alkali metal salts, and ammonium salts of these.

In view of providing favorable bond strength as a dental curable composition, preferred among these polymerizable monomers (A-2) having an acidic group are polymerizable monomers having a phosphoric acid group, or polymerizable monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-(meth)acryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is most preferred.

The polymerizable monomer (A-2) having an acidic group may be incorporated alone, or two or more thereof may be used in combination.

In view of adhesive properties to tooth structure, the content of polymerizable monomer (A-2) having an acidic group in a dental curable composition of the present invention is preferably 0 to 30 parts by mass, more preferably 0 to 20 parts by mass, even more preferably 0 to 15 parts by mass in total 100 parts by mass of polymerizable monomer (A).

### [Filler (B)]

It is required that a dental curable composition of the present invention comprise a filler (B), in order to adjust paste flowability and formability, and to increase the mechanical strength of the cured product. Examples of such fillers include inorganic fillers, organic fillers, and organic-inorganic composite fillers.

Examples of inorganic filler components include various types of glass (containing silica as a main component, and, optionally, oxides of elements such as heavy metals, boron, and aluminum). Examples of such glass include glass powders of common compositions, such as fused silica, quartz, soda-lime-silica glass, E glass, C glass, and borosilicate glass (PYREX^{®} glass); and dental glass powders such as barium glass (GM27884 and 8235 manufactured by SCHOTT, and E-2000 and E-3000 manufactured by ESSTECH), strontium·borosilicate glass (E-4000 manufactured by ESSTECH), lanthanum glass-ceramic (GM31684 manufactured by SCHOTT), and fluoroaluminosilicate glass (GM35429, G018-091, and G018-117 manufactured by SCHOTT). Other examples of inorganic filler components include various types of ceramics, composite oxides (such as silica-titania, and silica-zirconia), diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of strength and other properties, preferred among these are various types of glass, composite oxides (such as silica-titania, and silica-zirconia), core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

Examples of organic filler components include polymethyl methacrylate, polyethyl methacrylate, a methyl methacrylate-ethyl methacrylate copolymer, crosslinked polymethyl methacrylate, crosslinked polyethyl methacrylate, polyamide, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer.

The organic-inorganic composite filler is a filler obtained by adding a polymerizable monomer to the inorganic filler, polymerizing the mixture in paste form, and pulverizing the polymerized filler. The organic-inorganic composite filler may be, for example, a TMPT filler (a mixture of trimethylolpropane methacrylate and silica filler pulverized after polymerization).

The filler (B) may be spherical or irregular in shape, for example. In view of satisfying both paste flowability and formability, a dental curable composition of the present invention requires using both spherical filler (B-1) and irregularly shaped filler (B-2) as the filler (B).

### <Spherical Filler (B-1)>

The spherical filler (B-1) represents particles that appear round in shape when observed in a unit field of an electron micrograph, and that have an average uniformity of 0.6 or more as calculated by dividing the diameter of a particle perpendicular to its maximum diameter by the maximum diameter. In view of even superior sliding effect, the spherical filler (B-1) in a dental curable composition of the present invention has an average uniformity of preferably 0.7 or more, more preferably 0.8 or more.

The material of spherical filler (B-1) is not particularly limited because the spherical filler (B-1) can produce a sliding effect by being spherical, and mitigate interference between fillers. A certain embodiment is, for example, a dental curable composition in which the spherical filler (B-1) is an inorganic filler.

The spherical filler (B-1) used in the present invention has an average particle diameter of 0.01 to 1 µm, preferably 0.02 to 0.5 µm, more preferably 0.05 to 0.1 µm. When the average particle diameter is less than 0.01 µm, it leads to an increase in specific surface area, causing the paste to thicken, resulting in inadequate filler incorporation and reduced mechanical strength. Conversely, when the average particle diameter is more than 1 µm, the surface area of the spherical filler decreases, and the resultant cured product of the dental curable composition cannot exhibit high formability. In the present invention, the average particle diameter of filler (B) means the average particle diameter before surface treatment when the filler (B) is subjected to surface treatment.

The spherical filler (B-1) does not necessarily need to be produced or acquired as a singular particle, and may be one obtained by mixing two or more fillers of different average particle diameters or different components, provided that the average particle diameter of each filler falls within the foregoing ranges.

The spherical filler (B-1) used in the present invention needs to be a spherical filler hydrophobized with hydrophobizing agent (x-1).

The inclusion of a specific quantity of hydrophobized spherical filler (B-1) not only produces a sliding effect under external force but also diminishes the hydrophilic interaction between the polymerizable monomer and the filler, allowing the paste to maintain its flowability when subjected to external force, resulting in improvement of paste flowability. The hydrophobizing agent (x-1) will be described later.

### <Irregularly Shaped Filler (B-2)>

The irregularly shaped filler (B-2) used in the present invention has an average particle diameter of 0.5 to 6.0 µm. In view of mechanical strength and coating thickness, the average particle diameter of irregularly shaped filler (B-2) is preferably 1.0 to 5.0 µm, more preferably 1.5 to 3.0 µm. When the average particle diameter is less than 0.5 µm, the specific surface area of the irregularly shaped filler increases, resulting in reduced paste flowability. Conversely, when the average particle diameter is more than 6.0 µm, the dispersibility of the irregularly shaped filler in the dental curable composition deteriorates, and a uniform paste cannot be prepared.

The irregularly shaped filler (B-2) does not necessarily need to be produced or acquired as a singular particle, and may be one obtained by mixing two or more fillers of different average particle diameters or different components, provided that the average particle diameter of each filler falls within the foregoing ranges.

Incorporating a predetermined quantity of irregularly shaped filler (B-2) in total 100 parts by mass of filler (B) results in a cured product with outstanding mechanical strength and a paste that exhibits excellent formability.

Combining irregularly shaped filler (B-2) and spherical filler (B-1) also produces excellent flowability in the paste of a dental curable composition of the present invention.

### <Irregularly Shaped Fine Particulate Filler (B-3)>

In a dental curable composition of the present invention, the filler (B) comprises 0 to 10 parts by mass of irregularly shaped fine particulate filler (B-3) having an average particle diameter of less than 0.5 µm, in addition to the spherical filler (B-1) and irregularly shaped filler (B-2). The irregularly shaped fine particulate filler (B-3) serves as a thixotropic modifier, allowing for fine adjustments of properties such as flowability.

The irregularly shaped fine particulate filler (B-3) does not necessarily need to be produced or acquired as a singular particle, and may be one obtained by mixing two or more fillers of different average particle diameters or different components, provided that the average particle diameter of each filler falls within the foregoing ranges.

A dental curable composition of the present invention comprises 1 to 60 parts by mass of spherical filler (B-1), 40 to 99 parts by mass of irregularly shaped filler (B-2), and 0 to 10 parts by mass of irregularly shaped fine particulate filler (B-3) in total 100 parts by mass of filler (B). It is not possible to achieve excellent flowability and formability when the filler content deviates from these ranges.

The filler (B) comprises preferably 3 to 55 parts by mass of spherical filler (B-1), 54.9 to 96.9 parts by mass of irregularly shaped filler (B-2), and 0.1 to 8 parts by mass of irregularly shaped fine particulate filler (B-3), more preferably 5 to 50 parts by mass of spherical filler (B-1), 49.9 to 94.9 parts by mass of irregularly shaped filler (B-2), and 0.1 to 8 parts by mass of irregularly shaped fine particulate filler (B-3), even more preferably 10 to 30 parts by mass of spherical filler (B-1), 69 to 89 parts by mass of irregularly shaped filler (B-2), and 1 to 5 parts by mass of irregularly shaped fine particulate filler (B-3).

Preferably, the content of irregularly shaped filler (B-2) surpasses that of spherical filler (B-1) because it makes it easier to achieve excellent flowability and formability.

In view of paste flowability, formability, and the mechanical strength of the cured product, the content of the filler (B) in a dental curable composition of the present invention is preferably 50 to 2,000 parts by mass with respect to total 100 parts by mass of polymerizable monomer (A). In view of providing even superior effects, the content of filler (B) is more preferably 100 to 1,500 parts by mass, even more preferably150 to 1,000 parts by mass with respect to total 100 parts by mass of polymerizable monomer (A).

In view of producing excellent formability and providing outstanding mechanical strength in the cured product, it is preferable in certain embodiments that the content of the filler (B) in a dental curable composition of the present invention be 45 to 95 parts by mass, more preferably 55 to 92 parts by mass, even more preferably 60 to 90 parts by mass in total 100 parts by mass of the composition.

In this specification, the average particle diameter of filler can be determined using a laser diffraction scattering method or by observing particles with an electron microscope. Specifically, a laser diffraction scattering method is more convenient for the measurement of particles having a particle diameter of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi, Ltd.), and the size of particles (at least 200 particles) observed in a unit field of the micrograph may be measured using image-analyzing particle-size-distribution measurement software (Macview, manufactured by Mountech Co., Ltd.). Here, the particle diameter is determined as an arithmetic mean value of the maximum and minimum lengths of particles, and the average primary particle diameter is calculated from the number of particles and the particle diameter.

In a dental curable composition of the present invention, it is necessary to use a mixture or a combination of two or more fillers of different materials, different particle size distributions, and different forms. By combining two or more types of fillers, the fillers can interact with the polymerizable monomer or with the other fillers at an increased number of points, in addition to densely packing themselves.

Depending on the type of filler, it is also possible to control paste flowability in the presence or absence of a shear force. Different types of fillers may be incorporated within fillers of different particle sizes.

Particles other than fillers may be unintentionally present as impurities, as long as it does not hinder the effectiveness of the present invention.

Preferably, the filler (B) in the present invention is one that is surface-treated with a hydrophobizing agent to improve its dispersibility in the polymerizable monomer. Particularly, the spherical filler (B-1) needs to be hydrophobized with hydrophobizing agent (x-1).

In view of providing even superior properties including dispersibility in the polymerizable monomer and mechanical strength, the irregularly shaped filler (B-2) is preferably one that is hydrophobized with hydrophobizing agent (x-2).

The hydrophilic interaction with the polymerizable monomer can be diminished as long as the filler is hydrophobized (particularly, the spherical filler (B-1)). Accordingly, the hydrophobizing agent is not particularly limited, and any known surface treatment agent can be used as long as it is capable of imparting hydrophobicity.

The hydrophobizing agents (x-1) and (x-2) may be the same or different hydrophobizing agents.

The material of spherical filler (B-1) is not particularly limited because a dental curable composition of the present invention can produce the desired effect of hydrophobization as long as it comprises the spherical filler (B-1) with hydrophobic treatment.

In the present invention, the surface treatment agent used for surface treatment is not required to contain a surfactant. A certain embodiment is, for example, a dental curable composition in which the spherical filler (B-1) is hydrophobized with only the hydrophobizing agent (x-1).

Another embodiment is, for example, a dental curable composition in which the spherical filler (B-1) is not treated with a surfactant.

Yet another embodiment is, for example, a dental curable composition in which none of the fillers (B) is treated with a surfactant.

Still another embodiment is, for example, a dental curable composition that comprises a polymerizable monomer (A), a filler (B), and a polymerization initiator (C), without a surfactant.

Typical examples of hydrophobizing agent (x-1) include silane coupling agents, titanate-based coupling agents, aluminate-based coupling agents, and zirco-aluminate-based coupling agents. In view of enhancing the effectiveness of the present invention, particularly preferred is a treatment with a silane coupling agent. The hydrophobizing agent (x-1) may be used alone, or two or more thereof may be used in combination. In the case where two or more hydrophobizing agents (x-1) are used in combination, the resultant surface treatment layer may be a mixture of two or more surface treatment agents, or may be a multilayer structure with a plurality of surface treatment layers. The surface treatment method is not particularly limited, and may be any known method.

The silane coupling agent is not particularly limited, and known silane coupling agents may be used, as long as it can impart hydrophobicity. However, in view of enhanced effectiveness of the present invention, preferred for use is a silane coupling agent having a polymerizable group and represented by the general formula (1) below. By using a silane coupling agent having a polymerizable group and represented by the following general formula (1), the filler dispersibility improves, leading to enhanced paste flowability. Additionally, the mechanical strength of the cured product improves through polymerization of the surface treatment layer on the filler and each type of polymerizable monomer.

Y-SiRₙX₍₃₋ₙ₎ (1),

wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, n represents an integer of 0, 1, or 2, and R may be the same or different from each other when R is plural, and X may be the same or different from each other when X is plural.

The polymerizable group present in Y is not particularly limited, and may be, for example, a (meth)acryloyl group, a vinyl group, a mercapto group, a (meth)allyl group, or an epoxy group. In view of mechanical strength and other properties, preferred among these are (meth)acryloyl groups, more preferably methacryloyl groups.

When Y is a monovalent organic group having a polymerizable group, and the polymerizable group is bound to the organic group, the polymerizable group may be bound to the organic group either directly or via a divalent group containing a heteroatom such as an oxygen atom or nitrogen atom. For example, when the polymerizable group present in Y is a (meth)acryloyl group, Y may form a (meth)acryloyloxy group or a (meth)acrylamide group.

The number of polymerizable groups present in Y is not particularly limited, and is preferably 1 to 4, more preferably 1 or 2, even more preferably 1. When Y has a plurality of polymerizable groups, the polymerizable groups may be the same or different from each other.

Y may represent only polymerizable groups such as above, or may be a monovalent organic group having a polymerizable group. When Y is a monovalent organic group having a polymerizable group, Y may be a group in which the functional group and the organic group are bound to each other either directly or indirectly via a divalent group having a heteroatom such as an oxygen atom or nitrogen atom. The organic group is not particularly limited, and is, for example, an alkyl group having 1 to 20 carbon atoms, an aryl group having 6 to 18 carbon atoms, or an aralkyl group having 7 to 26 carbon atoms. For considerations such as further improvement of adhesive properties to both dental restoration materials and tooth structures, preferred among these are alkyl groups having 1 to 20 carbon atoms, more preferably alkyl groups having 3 to 11 carbon atoms, even more preferably alkyl groups having 5 to 11 carbon atoms. Examples of alkyl groups having 5 to 11 carbon atoms include n-pentyl groups, n-hexyl groups, n-octyl groups, and n-undecyl groups. Preferred are n-octyl groups and n-undecyl groups.

Examples of Y include (meth)acryloyloxymethyl groups, (meth)acryloyloxyethyl groups, γ-(meth)acryloyloxypropyl groups, γ-(meth)acryloyloxyoctyl groups, γ-(meth)acryloyloxyundecyl groups, γ-(meth)acrylamidepropyl groups, vinyl groups, (meth)allyl groups, and γ-glycidoxypropyl groups. Preferred are (meth)acryloyloxymethyl groups, (meth)acryloyloxyethyl groups, γ-(meth)acryloyloxypropyl groups, γ-(meth)acryloyloxyoctyl groups, and γ-(meth)acryloyloxyundecyl groups. More preferred are γ-(meth)acryloyloxyoctyl groups, and γ-(meth)acryloyloxyundecyl groups.

The alkyl group represented by R is not particularly limited, and may be, for example, an alkyl group having 1 to 5 carbon atoms. Specific examples include methyl groups, ethyl groups, n-propyl groups, isopropyl groups, n-butyl groups, and n-pentyl groups.

The aryl group represented by R is not particularly limited, and may be, for example, an aryl group having 6 to 10 carbon atoms. Specific examples include phenyl groups and naphthyl groups.

The aralkyl group represented by R is not particularly limited, and may be, for example, an aralkyl group having 7 to 12 carbon atoms. Specific examples include benzyl groups.

In view of mechanical strength and other properties, R is preferably an alkyl group, more preferably an alkyl group having 1 to 5 carbon atoms, even more preferably a methyl group.

The hydrolyzable group represented by X may be a group that, through hydrolysis, has the capability to form a silanol group with the silicon atom to which it is attached. Examples include alkoxy groups, acyloxy groups, siloxy groups, and halogen atoms.

The alkoxy group is not particularly limited, and may be, for example, an alkoxy group having 1 to 5 carbon atoms. Specific examples include methoxy groups, ethoxy groups, n-propoxy groups, isopropoxy groups, n-butoxy groups, and n-pentyloxy groups.

The acyloxy group is not particularly limited, and may be, for example, an acyloxy group having 1 to 5 carbon atoms. Specific examples include formyloxy groups, acetoxy groups, n-propionyloxy groups, isopropionyloxy groups, n-butanoyloxy groups, and n-pentanoyloxy groups.

The siloxy group is not particularly limited, and may be, for example, a trimethylsiloxy group.

The halogen atom is not particularly limited, and may be, for example, a chlorine atom or a bromine atom.

Preferably, X is an alkoxy group, more preferably an alkoxy group having 1 to 5 carbon atoms, even more preferably a methoxy group or an ethoxy group.

The symbol n represents an integer of 0 to 2. In view of properties such as the mechanical strength of the cured product of the dental curable composition, n is preferably 0 or 1. When n is 0 or 1, the multiple X may be the same or different from each other. When n is 2, the multiple R may be the same or different from each other.

Examples of the silane coupling agent include:
silane compounds containing a (meth)acryloyl group, such as (meth)acryloyloxymethyltrimethoxysilane, 2-(meth)acryloyloxyethyltrimethoxysilane, 3-(meth)acryloyloxypropyltrimethoxysilane, 4-(meth)acryloyloxybutyltrimethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11 -(meth)acryloyloxyundecyltrimethoxysilane, 11-(meth)acryloyloxyundecyldichloromethylsilane, 11-(meth)acryloyloxyundecyltrichlorosilane, 11-(meth)acryloyloxyundecyldimethoxymethylsilane, 12- (meth)acryloyloxydodecyltrimethoxysilane, 13- (meth)acryloyloxytridecyltrimethoxysilane, 3-(meth)acryloyloxypropylmethyldimethoxysilane, 3-(meth)acryloyloxypropylmethyldiethoxysilane, 3-(meth)acryloyloxypropylmethyldiisopropoxysilane, 3-(meth)acryloyloxypropylmethyldi(trimethylsiloxy)silane, 3-(meth)acryloyloxypropylmethyldi(hexylsiloxy)silane, 3-(meth)acryloyloxypropyltris(trimethylsiloxy)silane, (meth)acryloyloxy-2-(2-vinyloxyethoxy)ethylmethyldimethoxysilane, 6-(meth)acryloyloxyhexylmethyldimethoxysilane, (meth)acryloyloxy-p-phenylethylmethyldimethoxysilane, 6-(meth)acryloyloxyhexylmethyldiethoxysilane, 10-(meth)acryloyloxydecylmethyldimethoxysilane, 11-(meth)acryloyloxyundecylmethyldimethoxysilane, 11-(meth)acryloyloxyundecylmethyldiethoxysilane, 11-(meth)acryloyloxyundecylmethyldihexyloxysilane, 20-(meth)acryloyloxyeicosylmethyldimethoxysilane, 3-(meth)acryloyloxypropylphenyldimethoxysilane, 3-(meth)acryloyloxypropylmethyldichlorosilane, 11-(meth)acryloyloxyundecylmethyldichlorosilane, 11-(meth)acryloyloxyundecylethyldichlorosilane, 3-(meth)acryloyloxypropyldimethylmonomethoxysilane, 3-(meth)acryloyloxypropyldimethylmonoethoxysilane, 3-(meth)acryloyloxypropyldimethylmonoisopropoxysilane, 3-(meth)acryloyloxypropyldimethylmonotrimethylsiloxysilane, 3-(meth)acryloyloxypropyldimethylmonohexyloxysilane, (meth)acryloyloxy-2-(2-vinyloxyethoxy)ethyldimethylmonomethoxysilane, 6-(meth)acryloyloxyhexyldimethylmonomethoxysilane, (meth)acryloyloxy-p-phenylethyldimethylmonomethoxysilane, 6-(meth)acryloyloxyhexyldimethylmonoethoxysilane, 10-(meth)acryloyloxydecyldimethylmonomethoxysilane, 11-(meth)acryloyloxyundecyldimethylmonomethoxysilane, 11-(meth)acryloyloxyundecyldimethylmonoethoxysilane, 11-(meth)acryloyloxyundecyldimethylmonohexyloxysilane, 20-(meth)acryloyloxyeicosyldimethylmonomethoxysilane, 3-(meth)acryloyloxypropyldiphenylmonomethoxysilane, 3-(meth)acryloyloxypropyldimethylmonochlorosilane, 11-(meth)acryloyloxyundecyldimethylmonochlorosilane, and 11-(meth)acryloyloxyundecyldiethylmonochlorosilane;
silane compounds containing a vinyl group, such as vinylmethyldimethoxysilane, vinylmethyldiethoxysilane, vinylmethyldichlorosilane, vinylmethyldiacetoxysilane, vinylmethyldi(2-methoxyethoxy)silane, vinyldimethylmonomethoxysilane, vinyldimethylmonoethoxysilane, vinyldimethylmonochlorosilane, vinyldimethylmonoacetoxysilane, and vinyldimethylmono(2-methoxyethoxy)silane;
silane compounds containing an epoxy group, such as 3-glycidoxypropylmethyldimethoxysilane, 3-glycidoxypropylmethyldiethoxysilane, 3-glycidoxypropyldimethylmonomethoxysilane, and 3-glycidoxypropyldimethylmonoethoxysilane;
silane coupling agents having a polymerizable group represented by general formula (1), such as allylmethyldiethoxysilane, and silane compounds containing an allyldimethylmonoethoxysilane (meth)allyl group; and
silane coupling agents having no polymerizable group, such as hexaethyldisilazane, hexa n-propyldisilazane, hexaisopropyldisilazane, 1,1,2,2-tetramethyl-3,3-diethyldisilazane, 1,1,3,3-tetramethyldisilazane, 1,1,1,3,3,3-hexamethyldisilazane, and 1,1,1,3,3-pentamethyldisilazane.

The silane coupling agent may be a hydrolysate and/or condensate of these. The silane coupling agent may be used alone, or two or more thereof may be used in combination.

### <Hydrophobizing Agent (x-1) Used for Surface Treatment of Spherical Filler (B-1)>

In view of the density of the surface treatment layer, the hydrophobizing agent (x-1) used for the surface treatment of the spherical filler (B-1) is preferably a combination of two or more hydrophobizing agents (x-1), more preferably a silane coupling agent having a polymerizable group and represented by the general formula (1), in combination with a silane coupling agent having no polymerizable group, even more preferably a silane coupling agent having at least one polymerizable group selected from the group consisting of 3-methacryloyloxypropyltrimethoxysilane, 8-(meth)acryloyloxyoctyltrimethoxysilane, 11-(meth)acryloyloxyundecyltrimethoxysilane, and a hydrolysate of these, in combination with 1,1,1,3,3,3-hexamethyldisilazane. The hydrolysate can sustain the polymerization activity of the polymerizable group through the hydrolysis of its hydrolyzable group. The surface treatment layer formed on the filler surface can achieve higher density, and the present invention can exhibit even greater effectiveness when two or more hydrophobizing agents (x-1) are used in combination, particularly when combining treatment agents differing in molecular bulk, such as when the silane coupling agent having a polymerizable group and represented by the general formula (1) is used with a silane coupling agent having no polymerizable group.

### <Hydrophobizing Agent (x-2) used for Surface Treatment of Irregularly Shaped Filler (B-2)>

Examples of the hydrophobizing agent (x-2) used for the surface treatment of irregularly shaped filler (B-2) include the same hydrophobizing agents exemplified for the hydrophobizing agent (x-1) used for the surface treatment of spherical filler (B-1). The hydrophobizing agent (x-2) may be used alone, or two or more thereof may be used in combination. In view of properties such as the mechanical strength of the cured product and the dispersibility of the filler, the hydrophobizing agent (x-2) is preferably a silane coupling agent having at least one polymerizable group selected from the group consisting of 3-methacryloyloxypropyltrimethoxysilane, 8-(meth)acryloyloxyoctyltrimethoxysilane, 11-(meth)acryloyloxyundecyltrimethoxysilane, and a hydrolysate of these, more preferably a silane coupling agent having at least one polymerizable group selected from the group consisting of 8-(meth)acryloyloxyoctyltrimethoxysilane, 11-(meth)acryloyloxyundecyltrimethoxysilane, and a hydrolysate of these, even more preferably 8-(meth)acryloyloxyoctyltrimethoxysilane, or a hydrolysate thereof.

### [Polymerization Initiator (C)]

The polymerization initiator (C) is classified into photopolymerization initiator (C-1) and chemical polymerization initiator (C-2). It is required that a dental curable composition of the present invention comprise a photopolymerization initiator (C-1) as a polymerization initiator (C). The photopolymerization initiator (C-1) may be used alone, or may be used with a chemical polymerization initiator (C-2).

### <Photopolymerization Initiator (C-1)>

The photopolymerization initiator (C-1) is not particularly limited, and may be any known photopolymerization initiator. Examples of known photopolymerization initiators include α-diketones, ketals, thioxanthones, (bis)acylphosphine oxides, and α-aminoacetophenones.

Examples of the α-diketones include dl-camphorquinone (commonly known as CQ), benzyl, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of the (bis)acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphine oxide, 2,4,6-trimethylbenzoylethoxyphenylphosphine oxide, and the water-soluble acylphosphine oxide compounds disclosed in JP H03-57916 B.

Examples of the α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

In view of properties such as curability, the photopolymerization initiator (C-1) is preferably an α-diketone or an acylphosphine oxide, more preferably an α-diketone, even more preferably a camphorquinone.

The photopolymerization initiator (C-1) may be used alone, or two or more thereof may be used in combination. In view of curability and storage stability, the content of photopolymerization initiator (C-1) is preferably 0.01 to 10 parts by mass, more preferably 0.05 to 5 parts by mass, even more preferably 0.1 to 3 parts by mass with respect to total 100 parts by mass of polymerizable monomer (A).

### <Chemical Polymerization Initiator (C-2)>

The chemical polymerization initiator (C-2) is not particularly limited, and may be any known chemical polymerization initiator.

With a dental curable composition of the present invention comprising a chemical polymerization initiator (C-2), the resultant cured product can achieve even greater mechanical strength.

Preferably, the chemical polymerization initiator (C-2) comprises organic peroxides and/or inorganic peroxides.

Examples of the organic peroxides include hydroperoxides, peroxyesters, ketone peroxides, peroxyketals, dialkyl peroxides, diacyl peroxides, and peroxydicarbonates. Preferred among these are hydroperoxides and peroxyesters.

Examples of the hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide.

The peroxyesters may be any known peroxyesters, provided that the peroxy group has an acyl group at one end, and a hydrocarbon group or a similar group at the other end. Specific examples include α,α-bis(neodecanoylperoxy)diisopropylbenzene, cumylperoxyneodecanoate, 1,1,3,3-tetramethylbutylperoxyneodecanoate, 1-cyclohexyl-1-methylethylperoxyneodecanoate, t-hexylperoxyneodecanoate, t-butylperoxyneodecanoate, t-hexylperoxypivalate, t-butylperoxypivalate, 1,1,3,3-tetramethylbutylperoxy-2-ethylhexanoate, 2,5-dimethyl-2,5-bis(2-ethylhexanoylperoxy)hexane, 1-cyclohexyl-1-methylethyl peroxy-2-ethylhexanoate, t-hexylperoxy-2-ethylhexanoate, t-butylperoxy-2-ethylhexanoate, t-butylperoxyisobutyrate, t-hexylperoxyisopropyl monocarbonate, t-butylperoxymaleic acid, t-butylperoxy-3,5,5-trimethylhexanoate, t-butylperoxylaurate, 2,5-dimethyl-2,5-bis(m-toluoylperoxy)hexane, t-butylperoxyisopropyl monocarbonate, t-butylperoxy-2-ethylhexyl monocarbonate, t-hexylperoxybenzoate, 2,5-dimethyl-2,5-bis(benzoylperoxy)hexane, t-butylperoxyacetate, t-butylperoxy-m-toluoylbenzoate, t-butylperoxybenzoate, and bis(t-butylperoxy)isophthalate. In view of storage stability and reactivity, preferred among these are t-butylperoxymaleic acid, t-butylperoxy-3,5,5-trimethylhexanoate, t-butylperoxybenzoate, t-butylperoxyisopropyl monocarbonate, t-butylperoxy-2-ethylhexyl monocarbonate, and t-butylperoxyacetate, more preferably t-butylperoxybenzoate.

Examples of the ketone peroxides include methyl ethyl ketone peroxide, cyclohexanone peroxide, methyl cyclohexanone peroxide, methyl acetoacetate peroxide, and acetyl acetone peroxide.

Examples of the peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl-4,4-bis(t-butylperoxy)valerate, and 2,2-bis(4,4-di-t-butylperoxycyclohexyl)propane.

Examples of the dialkyl peroxides include α,α-bis(t-butylperoxy)diisopropylbenzene, dicumyl peroxide, 2,5-dimethyl-2,5-bis(t-butylperoxy)hexane, t-butyl cumylperoxide, di-t-butyl peroxide, and 2,5-dimethyl-2,5-bis(t-butylperoxy)3-hexyne.

Examples of the diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of the peroxydicarbonates include di-n-propylperoxy dicarbonate, diisopropylperoxy dicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of the inorganic peroxides include peroxydisulfates and peroxydiphosphates. In view of reactivity, peroxydisulfates are preferred. Examples of the peroxydisulfates include sodium peroxydisulfate, potassium peroxydisulfate, aluminum peroxydisulfate, and ammonium peroxydisulfate. Preferred among these are sodium peroxydisulfate, potassium peroxydisulfate, and ammonium peroxydisulfate.

### <Chemical Polymerization Initiator (C-2)>

The chemical polymerization initiator (C-2) may be incorporated alone, or two or more thereof may be used in combination. In view of bond strength to the adherend and working time margin, the content of chemical polymerization initiator (C-2) is preferably 0.001 to 20 parts by mass, more preferably 0.005 to 10 parts by mass, even more preferably 0.025 to 5 parts by mass with respect to total 100 parts by mass of polymerizable monomer (A). It is easier to provide adequate adhesive properties when the content of chemical polymerization initiator (C-2) is 0.001 parts or more by mass, whereas a content of 20 parts or less by mass facilitates maintaining the working time margin.

### [Polymerization Accelerator (D)]

A dental curable composition of the present invention may comprise a polymerization accelerator (D). The polymerization accelerator (D) is a compound that accelerates a reaction in conjunction with the polymerization initiator (C). The polymerization accelerator (D) is classified into photopolymerization accelerator (D-1) and chemical polymerization accelerator (D-2).

The polymerization accelerator (D) may comprise either one of photopolymerization accelerator (D-1) and chemical polymerization accelerator (D-2), or may comprise both photopolymerization accelerator (D-1) and chemical polymerization accelerator (D-2).

### <Photopolymerization Accelerator (D-1)>

The photopolymerization accelerator (D-1) is not particularly limited, and may be any known photopolymerization accelerator.

Examples of the photopolymerization accelerator (D-1) used in a dental curable composition of the present invention include aldehydes, thiol compounds, aminobenzoic acid ester compounds, and amine-based reducing agents.

Examples of the aldehydes include terephthalaldehyde, and benzaldehyde derivatives. Examples of the benzaldehyde derivatives include dimethylaminobenzaldehyde, p-methoxybenzaldehyde, p-ethoxybenzaldehyde, and p-n-octyloxybenzaldehyde.

Examples of the thiol compounds include 2-mercaptobenzoxazole, decanethiol, 3-mercaptopropyltrimethoxysilane, and thiobenzoic acid.

Examples of the aminobenzoic acid ester compounds include ethyl 4-(N,N-dimethylamino)benzoate, methyl 4-(N,N-dimethylamino)benzoate, n-butoxyethyl 4-N,N-(dimethylamino)benzoate, 2-[(meth)acryloyloxy]ethyl 4-(N,N-dimethylamino)benzoate, 4-(N,N-dimethylamino)benzophenone, and n-butyl 4-(N,N-dimethylamino)benzoate.

The amine-based reducing agents can be broadly classified into aromatic amines and aliphatic amines. In the present invention, the amine-based reducing agents may be aromatic amines or aliphatic amines.

The aromatic amines may be, for example, known aromatic secondary amines or aromatic tertiary amines. Examples of the aromatic secondary amines or aromatic tertiary amines include N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

Examples of the aliphatic amines include aliphatic primary amines such as n-butylamine, n-hexylamine, and n-octylamine; aliphatic secondary amines such as diisopropylamine, dibutylamine, and N-methylethanolamine; and aliphatic tertiary amines such as N-methyldiethanolamine, N-ethyldiethanolamine, N-n-butyldiethanolamine, N-lauryldiethanolamine, 2-(dimethylamino)ethyl (meth)acrylate, N-methyldiethanolamine di(meth)acrylate, N-ethyldiethanolamine di(meth)acrylate, triethanolamine tri(meth)acrylate, triethanolamine, trimethylamine, triethylamine, and tributylamine.

In view of curability and storage stability, preferred among these photopolymerization accelerators are aminobenzoic acid ester compounds, and aliphatic tertiary amines, more preferably ethyl 4-(N,N-dimethylamino)benzoate, N-methyldiethanolamine, and triethanolamine.

The photopolymerization accelerator (D-1) may be incorporated alone, or two or more thereof may be used in combination.

In view of curability and storage stability, the content of photopolymerization accelerator (D-1) is preferably 0.01 to 10 parts by mass, more preferably 0.05 to 5 parts by mass, even more preferably 0.1 to 3 parts by mass with respect to total 100 parts by mass of polymerizable monomer (A). It is easier to provide adequate adhesive properties when the content of photopolymerization accelerator (D-1) is 0.01 parts or more by mass, whereas a content of 10 parts or less by mass facilitates maintaining the working time margin.

### <Chemical Polymerization Accelerator (D-2)>

The chemical polymerization accelerator (D-2) used in a dental curable composition of the present invention can be broadly classified into chemical polymerization accelerator (D-2a), which is suitably used when the polymerizable monomer (A) does not comprise a polymerizable monomer (A-2) having an acidic group, and chemical polymerization accelerator (D-2b), which is suitably used when the polymerizable monomer (A) comprises a polymerizable monomer (A-2) having an acidic group.

The chemical polymerization accelerator (D-2) may comprise either one or both of chemical polymerization accelerator (D-2a), which is suitably used when the polymerizable monomer (A) does not comprise a polymerizable monomer (A-2) having an acidic group, and chemical polymerization accelerator (D-2b), which is suitably used when the polymerizable monomer (A) comprises a polymerizable monomer (A-2) having an acidic group.

Note that copper compounds can be suitably used for both situations where the polymerizable monomer (A) comprises or does not comprise a polymerizable monomer (A-2) having an acidic group. Accordingly, copper compounds are presented as examples for both situations.

### · Chemical Polymerization Accelerator (D-2a)

Examples of the chemical polymerization accelerator (D-2a) in the dental curable composition include aromatic amines having no electron withdrawing group on the aromatic ring, Period 4 transition metal compounds, transition metal compounds outside of Period 4, and thiourea compounds. Specific examples are as follows.

Examples of aromatic amines having no electron withdrawing group on the aromatic ring include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

The Period 4 transition metal compounds may be any of vanadium compounds, copper compounds, and Period 4 transition metal compounds excluding vanadium and copper (hereinafter, also referred to simply as "alternative Period 4 transition metal compounds"). In view of polymerization accelerating effect, preferred as chemical polymerization accelerator (D-2a) in the present invention are vanadium compounds and copper compounds.

Examples of the vanadium compounds include vanadium acetylacetonate, vanadyl acetylacetonate, vanadyl stearate, vanadium naphthenate, vanadium benzoylacetonate, vanadyl oxalate, bis(maltolato)oxovanadium(IV), oxobis(1-phenyl-1,3-butanedionate)vanadium(IV), vanadium(V) oxytriisopropoxide, ammonium metavanadate, sodium metavanadate, vanadium(V) oxide, vanadium(IV) oxide, and vanadyl(IV) sulfate.

In view of properties such as the solubility in solvent, preferred are vanadium acetylacetonate, vanadyl acetylacetonate, and bis(maltolato)oxovanadium(IV), more preferably vanadyl acetylacetonate and bis(maltolato)oxovanadium(IV).

Preferably, the copper compounds are, for example, compounds that are soluble in radical polymerizable monomers.

Examples of the copper compounds include copper(II) carboxylates, copper(II) β-diketones, copper(II) β-ketoesters, copper alkoxides, copper dithiocarbamates, salts of copper and inorganic acids, copper procetonate, and copper complexes.

Examples of the copper(II) carboxylates include copper(II) acetate, copper(II) benzoate, copper(II) isobutyrate, copper(II) gluconate, copper(II) citrate, copper(II) phthalate, copper(II) tartarate, copper(II) oleate, copper(II) octylate, copper(II) octenate, copper(II) naphthenate, copper(II) methacrylate, copper(II) acrylate, and copper 4-cyclohexylbutyrate.

Examples of the copper(II) β-diketones include copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate.

Examples of the copper(II) β-ketoesters include copper(II) ethylacetoacetate. Examples of the copper alkoxides include copper(II) methoxide, copper(II) ethoxide, copper(II) isopropoxide, copper(II) 2-(2-butoxyethoxy)ethoxide, and copper(II) 2-(2-methoxyethoxy)ethoxide.

Examples of the copper dithiocarbamates include copper(II) dimethyldithiocarbamate.

Examples of the salts of copper and inorganic acids include copper nitrate, and copper chloride.

In view of solubility and reactivity to radical polymerizable monomers, preferred are copper(II) carboxylates, copper(II) β-diketones, and copper(II) β-ketoesters, more preferably copper(II) acetate, and copper(II) acetylacetonate.

Examples of the alternative Period 4 transition metal compounds include scandium isopropoxide, iron(III) ethoxide, titanium methoxide, titanium ethoxide, titanium isopropoxide, titanium butoxide, titanium hydroxide, and titanium fluoride.

In view of enhanced polymerization accelerating effect, preferred among the Period 4 transition metal compounds are vanadyl(IV) acetylacetonate, and bis(maltolato)oxovanadium(IV), more preferably vanadyl(IV) acetylacetonate.

Examples of the transition metal compounds outside of Period 4 include strontium carbonate, strontium hydroxide, strontium ethoxide, tin(II) methoxide, indium ethoxide, actinium ethoxide, yttrium isopropoxide, lanthanum methoxide, lanthanum ethoxide, lanthanum isopropoxide, lanthanum butoxide, lanthanum hydroxide, lanthanum carbonate, lanthanum fluoride, cerium isopropoxide, praseodymium isopropoxide, promethium isopropoxide, neodymium isopropoxide, samarium isopropoxide, europium isopropoxide, gadolinium isopropoxide, terbium ethoxide, terbium methoxide, dysprosium isopropoxide, holmium isopropoxide, erbium isopropoxide, thulium isopropoxide, ytterbium isopropoxide, zirconium ethoxide, zirconium isopropoxide, zirconium butoxide, tungsten(IV) methoxide, tungsten(IV) isopropoxide, and tungsten(IV) butoxide.

Examples of the thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, (6-methyl-pyridin-2-yl)thiourea, and 4,4-dimethylethylenethiourea. In view of properties such as the solubility in solvent and storage stability, preferred are 1-(2-pyridyl)-2-thiourea, (6-methyl-pyridin-2-yl)thiourea, and 4,4-dimethylethylenethiourea.

The chemical polymerization accelerator (D-2a) may be incorporated alone, or two or more thereof may be used in combination. In view of curability and storage stability, the content of chemical polymerization accelerator (D-2a) is preferably 0.001 to 20 parts by mass, more preferably 0.005 to 10 parts by mass, even more preferably 0.025 to 5 parts by mass with respect to total 100 parts by mass of polymerizable monomer (A).

### · Chemical Polymerization Accelerator (D-2b)

Examples of the chemical polymerization accelerator (D-2b) in the dental curable composition include copper compounds, aromatic sulfinic acids and salts thereof, benzotriazole compounds, benzoimidazole compounds, bromides, and sulfur-containing reducing inorganic compounds. Specific examples are as follows.

Preferably, the copper compounds are compounds that are soluble in the polymerizable monomer components.

Examples of the copper compounds include copper(II) carboxylates, copper(II) β-diketones, copper(II) β-ketoesters, copper alkoxides, copper dithiocarbamates, salts of copper and inorganic acids, copper procetonate, and copper complexes.

Specific examples of the copper(II) carboxylates, copper(II) β-diketones, copper(II) β-ketoesters, copper alkoxides, copper dithiocarbamates, and salts of copper and inorganic acids include those exemplified for (D-2a).

In view of solubility and reactivity to the polymerizable monomers, preferred are copper(II) carboxylates, copper(II) β-diketones, and copper(II) β-ketoesters, more preferably copper(II) acetate, and copper(II) acetylacetonate.

Examples of the aromatic sulfinic acids and salts thereof include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid, and lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of these.

In view of polymerizability and storage stability of the composition, preferred among these are lithium salts, sodium salts, potassium salts, magnesium salts, and calcium salts of 2,4,6-trimethylbenzenesulfinic acid and 2,4,6-triisopropylbenzenesulfinic acid, more preferably lithium salts, sodium salts, potassium salts, magnesium salts, and calcium salts of 2,4,6-triisopropylbenzenesulfinic acid.

Preferably, the aromatic sulfinic acids and salts thereof are at least partially dispersed in powder form within the composition. With the aromatic sulfinic acids and salts thereof being dispersed in powder form, it is easier to provide a longer working time margin in a dental curable composition of the present invention, and, when the composition is applied to moist materials such as the tooth structure, the aromatic sulfinic acids and salts thereof dissolve in the water on the surface of moist material, making it possible to more greatly enhance polymerization both at the bonding interface and within the resin-impregnated layer. When the aromatic sulfinic acids and salts thereof are dispersed in powder form, it is preferable that the aromatic sulfinic acids and salts thereof have a solubility in water of 1 mg/100 mL or more at ordinary temperature (25°C). A solubility of 1 mg/100 mL or more facilitates sufficient dissolution of aromatic sulfinic acids and salts thereof in the water on moist materials at the bonding interface when a dental curable composition of the present invention is applied to moist materials, facilitating the effect of dispersing powder.

In view of resistance to settling, the aromatic sulfinic acids and salts thereof have an average particle diameter of preferably 500 µm or less, more preferably 100 µm or less, even more preferably 50 µm or less. In view of preventing excessive specific surface area in the powder and facilitating in maintaining good ease of handling for the dental curable composition, the average particle diameter is preferably 0.01 µm or more. That is, when dispersed as powder, the aromatic sulfinic acids and salts thereof have an average particle diameter ranging from preferably 0.01 to 500 µm, more preferably from 0.01 to 100 µm, even more preferably from 0.01 to 50 µm. The average particle diameter can be measured using the same method as that for the average particle diameter of filler (B).

The shape of the aromatic sulfinic acids and salts thereof dispersed in powder form is not particularly limited, and may be any of various shapes such as spherical, needle-shape, plate-like, and crushed-shape. Fine powders of aromatic sulfinic acid salts can be prepared using known methods such as pulverization and freeze drying.

In view of working time margin and adhesive properties, the content of the aromatic sulfinic acids and salts thereof is preferably 0.1 to 5 parts by mass, more preferably 0.2 to 4 parts by mass, even more preferably 0.5 to 3 parts by mass with respect to total 100 parts by mass of polymerizable monomer (A) in an adhesive composition of the present invention. A content of less than 0.1 parts by mass may cause a decrease in adhesive properties, whereas a content of more than 5 parts by mass may result in shorter working time margins.

The benzotriazole compounds and benzoimidazole compounds are represented by the following general formulae (2) and (3), respectively.

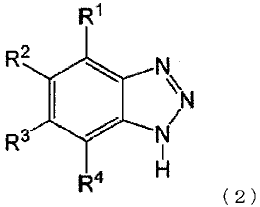

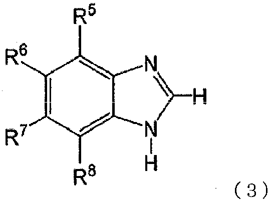

In the general formulae (2) and (3), R¹ to R⁸ each independently represent a hydrogen atom, a hydroxyl group, an alkyl group, an aryl group, an alkoxy group, an alkenyl group, an aralkyl group, or a halogen atom.

The alkyl groups represented by R¹ to R⁸ may be linear, branched, or cyclic, and are preferably ones having 1 to 10 carbon atoms. Examples include methyl groups, ethyl groups, n-propyl groups, isopropyl groups, cyclopropyl groups, n-butyl groups, isobutyl groups, sec-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups. Particularly preferred among these are methyl groups and ethyl groups.

The aryl groups represented by R¹ to R⁸ are preferably ones having 6 to 10 carbon atoms. Examples include phenyl groups, naphthyl groups, and anthryl groups.

The alkoxy groups represented by R¹ to R⁸ may be linear, branched, or cyclic, and are preferably ones having 1 to 8 carbon atoms. Examples include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkenyl groups represented by R¹ to R⁸ may be linear, branched, or cyclic, and are preferably ones having 1 to 6 carbon atoms. Examples include vinyl groups, allyl groups, methylvinyl groups, propenyl groups, butenyl groups, pentenyl groups, hexenyl groups, cyclopropenyl groups, cyclobutenyl groups, cyclopentenyl groups, and cyclohexenyl groups.

The aralkyl groups represented by R¹ to R⁸ are preferably alkyl groups (particularly, C1 to C10 alkyl groups) substituted with aryl groups (particularly, C6 to C10 aryl groups). Examples include benzyl groups.

Examples of the halogen atoms represented by R¹ to R⁸ include a fluorine atom, a chlorine atom, a bromine atom, and an iodine atom.

R¹ to R⁸ are preferably hydrogen atoms or methyl groups.

Only one type of benzotriazole compound or benzoimidazole compound may be used alone, or more than one type of benzotriazole compound or benzoimidazole compound may be used in combination. Examples of the benzotriazole compounds and benzoimidazole compounds include 1H-benzotriazole, 5-methyl-1H-benzotriazole, 5,6-dimethyl-1H-benzotriazole, benzoimidazole, 5-methylbenzoimidazole, and 5,6-dimethylbenzoimidazole. In view of working time margin, preferred are 1H-benzotriazole, and 5-methyl-1H-benzotriazole.

Examples of the bromides include zinc bromide, potassium bromide, sodium bromide, calcium bromide, indium bromide, ammonium bromide, and tetrabutylammonium bromide. Particularly preferred among these are zinc bromide, ammonium bromide, and tetrabutylammonium bromide.

Examples of the sulfur-containing reducing inorganic compounds include sulfites, bisulfites, pyrosulfites, thiosulfates, thionates, and dithionites. Preferred among these are sulfites and bisulfites. Specific examples include sodium sulfite, potassium sulfite, calcium sulfite, ammonium sulfite, sodium bisulfite, and potassium bisulfite.

Preferably, the sulfur-containing reducing inorganic compound are at least partially dispersed in powder form within the composition.

With the sulfur-containing reducing inorganic compound being dispersed in powder form, it is easier to provide a longer working time margin in a dental curable composition of the present invention, and, when the composition is applied to the tooth structure, the sulfur-containing reducing inorganic compounds dissolve in the water on the surface of tooth structure, making it possible to more greatly enhance polymerization both at the bonding interface and within the resin-impregnated layer.

When the sulfur-containing reducing inorganic compounds are dispersed in powder form, it is preferable that the sulfur-containing reducing inorganic compounds have a solubility in water of 1 mg/100 mL or more at ordinary temperature (25°C). A solubility of 1 mg/100 mL or more facilitates sufficient dissolution of sulfur-containing reducing inorganic compound in the water on tooth structure at the bonding interface when a dental curable composition of the present invention is applied to tooth structure, facilitating the effect of dispersing powder. In view of resistance to settling, the sulfur-containing reducing inorganic compounds have an average particle diameter of preferably 500 µm or less, more preferably 100 µm or less, even more preferably 50 µm or less.

In view of preventing excessive specific surface area in the powder and facilitating in maintaining good ease of handling for the dental curable composition, the average particle diameter is preferably 0.01 µm or more. That is, when dispersed as powder, the average particle diameter ranges preferably from 0.01 to 500 µm, more preferably from 0.01 to 100 µm, even more preferably from 0.01 to 50 µm.

The average particle diameter can be measured using the same method as that for the average particle diameter of filler (B).

The shape of the sulfur-containing reducing inorganic compounds dispersed in powder form is not particularly limited, and may be any of various shapes such as spherical, needle-shape, plate-like, and crushed-shape. Fine powders of sulfur-containing reducing inorganic compounds can be prepared using known methods such as pulverization and freeze drying.

The chemical polymerization accelerator (D-2b) may be incorporated alone, or two or more thereof may be used in combination.

In view of curability and storage stability, the content of chemical polymerization accelerator (D-2b) is preferably 0.001 to 40 parts by mass, more preferably 0.005 to 20 parts by mass, even more preferably 0.025 to 10 parts by mass with respect to total 100 parts by mass of polymerizable monomer (A).

In a dental curable composition of the present invention, it is also possible to incorporate known additives in appropriate combinations, for example, such as viscosity adjusters, thickeners, polymerization inhibitors, polymerization adjusters, ultraviolet absorbers, water, organic solvents, dyes, pigments, antimicrobial agents, acid amplifiers, base amplifiers, inorganic or organic acids, and hydrophobizing agents, depending on intended use.

Examples of the polymerization inhibitors include hydroquinone, hydroquinone monomethyl ether, dibutyl hydroquinone, dibutyl hydroquinone monomethyl ether, t-butyl catechol, 2-t-butyl-4,6-dimethylphenol, 2,6-di-t-butylphenol, and 2,6-di-t-butyl-4-methylphenol.

When a dental curable composition of the present invention comprises a chemical polymerization initiator (C-2) and a chemical polymerization accelerator (D-2), it is preferable in view of storage stability that the composition be packaged into a product form with two, three, or more separate packs to prevent reactions among its components prior to use. In this case, it is preferable to formulate the chemical polymerization initiator (C-2) separately from the thiourea compounds or aromatic sulfinic acids and salts thereof.

In the case of a two-pack type dental curable composition, it is preferable to incorporate the polymerizable monomer (A) and the filler (B) in both the first and second agents to prepare a two-paste composition.

A certain embodiment is, for example, a dental curable composition that comprises a first agent and a second agent, and in which the first agent comprises a polymerizable monomer (A), a filler (B), and a chemical polymerization accelerator (D-2), and the second agent comprises a polymerizable monomer (A), a filler (B), and a chemical polymerization initiator (C-2).

In such a dental curable composition comprising a first and a second agent, the combination of each component of the filler (B) (spherical filler (B-1), irregularly shaped filler (B-2), and irregularly shaped fine particulate filler (B-3)) is not particularly limited, as long as these are mixed at the time of use to attain the desired content and achieve the intended effect of the present invention.

A certain embodiment is, for example, a dental curable composition in which the first agent comprises a spherical filler (B-1) and an irregularly shaped filler (B-2), and the second agent comprises a spherical filler (B-1) and an irregularly shaped filler (B-2).

Another embodiment is, for example, a dental curable composition in which the first agent comprises an irregularly shaped filler (B-2), and the second agent comprises a spherical filler (B-1) and an irregularly shaped filler (B-2).

Yet another embodiment is, for example, a dental curable composition in which the first agent comprises a spherical filler (B-1) and an irregularly shaped filler (B-2), and the second agent comprises an irregularly shaped filler (B-2).

In any of the embodiments above, the first agent and/or the second agent may comprise an irregularly shaped fine particulate filler (B-3).

A dental curable composition of the present invention exhibits high levels of both paste flowability and formability. This makes a dental curable composition of the present invention usable for dental treatments in a variety of applications, for example, such as dental cements, self-adhesive dental cements, dental abutment construction materials, pit and fissure sealants, loose tooth fixing materials, and orthodontic bonding materials. A dental curable composition of the present invention is particularly suited as dental cements, self-adhesive dental cements, or dental abutment construction materials. The following describes specific embodiments of applications of the dental curable composition.

### <Dental Cement>

A preferred embodiment of a dental curable composition of the present invention is, for example, a dental cement. Preferred examples of the dental cement include dental resin cements, dental glass ionomer cements, and dental resin-reinforced glass ionomer cements. The dental cements may be used with pretreatment materials such as self-etching primers.

When a dental curable composition of the present invention is used as a dental cement, it is preferable that the composition comprise a polymerizable monomer (A), a filler (B), a polymerization initiator (C), and a polymerization accelerator (D), and that the polymerization initiator (C) be a combination of photopolymerization initiator (C-1) and chemical polymerization initiator (C-2).

Concerning the content of each component in the dental cement, it is preferable to comprise less than 15 parts by mass of hydrophilic polymerizable monomer (A-1a), 85 to 100 parts by mass of hydrophobic polymerizable monomer (A-1b), and 0 to 15 parts by mass of polymerizable monomer (A-2) having an acidic group, more preferably 1 to 10 parts by mass of hydrophilic polymerizable monomer (A-1a), 90 to 99 parts by mass of hydrophobic polymerizable monomer (A-1b), and 0 to 10 parts by mass of polymerizable monomer (A-2) having an acidic group, even more preferably 2 to 5 parts by mass of hydrophilic polymerizable monomer (A-1a), 95 to 98 parts by mass of hydrophobic polymerizable monomer (A-1b), and 0 to 3 parts by mass of polymerizable monomer (A-2) having an acidic group in total 100 parts by mass of the polymerizable monomer (A) in the dental curable composition. It is also preferable to comprise 50 to 2,000 parts by mass of filler (B), 0.001 to 20 parts by mass of polymerization initiator (C), and 0.001 to 40 parts by mass of polymerization accelerator (D), more preferably 100 to 1,500 parts by mass of filler (B), 0.005 to 10 parts by mass of polymerization initiator (C), and 0.005 to 20 parts by mass of polymerization accelerator (D) with respect to total 100 parts by mass of polymerizable monomer (A). The dental curable composition, when used as a dental cement, is not required to comprise a hydrophilic polymerizable monomer (A-1a). When used as a type of dental cement used with a pretreatment material, the dental curable composition is not required to comprise a polymerizable monomer (A-2) having an acidic group.

### <Self-Adhesive Dental Cement>

A preferred embodiment of a dental curable composition of the present invention is, for example, a self-adhesive dental cement, an embodiment of dental cement that does not use pretreatment materials such as primers. When a dental curable composition of the present invention is used as a self-adhesive dental cement, it is preferable that the composition comprise a polymerizable monomer (A), a filler (B), a polymerization initiator (C), and a polymerization accelerator (D), and that the polymerizable monomer (A) comprise a hydrophilic polymerizable monomer (A-1a), a hydrophobic polymerizable monomer (A-1b), and a polymerizable monomer (A-2) having an acidic group. It is also preferable that the polymerization initiator (C) be a combination of photopolymerization initiator (C-1) and chemical polymerization initiator (C-2). When used as a self-adhesive dental cement, a dental curable composition of the present invention may use a pretreatment material. However, use of pretreatment materials is not necessary because the self-adhesive properties of the composition eliminate the necessity for pretreatment materials.

Concerning the content of each component in the self-adhesive dental cement, it is preferable to comprise 15 parts or less by mass of hydrophilic polymerizable monomer (A-1a), 84 to 99 parts by mass of hydrophobic polymerizable monomer (A-1b), and 1 to 16 parts by mass of polymerizable monomer (A-2) having an acidic group, more preferably 1 to 10 parts by mass of hydrophilic polymerizable monomer (A-1a), 89 to 98 parts by mass of hydrophobic polymerizable monomer (A-1b), and 1 to 10 parts by mass of polymerizable monomer (A-2) having an acidic group, even more preferably 2 to 5 parts by mass of hydrophilic polymerizable monomer (A-1a), 94 to 97 parts by mass of hydrophobic polymerizable monomer (A-1b), and 1 to 4 parts by mass of polymerizable monomer (A-2) having an acidic group in total 100 parts by mass of the polymerizable monomer (A) in the dental curable composition. It is also preferable to comprise 50 to 2,000 parts by mass of filler (B), 0.001 to 20 parts by mass of polymerization initiator (C), and 0.001 to 40 parts by mass of polymerization accelerator (D), more preferably 100 to 1,000 parts by mass of filler (B), 0.005 to 10 parts by mass of polymerization initiator (C), and 0.005 to 20 parts by mass of polymerization accelerator (D) with respect to total 100 parts by mass of polymerizable monomer (A). The dental curable composition, when used as a self-adhesive dental cement, is not required to comprise a hydrophilic polymerizable monomer (A-1a).

### <Dental Abutment Construction Material>

A preferred embodiment of a dental curable composition of the present invention is, for example, a dental abutment construction material. The dental abutment construction material may use pretreatment materials such as self-etching primers.

When a dental curable composition of the present invention is used as a dental cement, it is preferable that the dental curable composition comprise a polymerizable monomer (A), a filler (B), a polymerization initiator (C), and a polymerization accelerator (D), and that the filler (B) comprise a spherical filler (B-1) with an average particle diameter of 0.01 µm to 1 µm treated with a hydrophobizing agent (x-1), an irregularly shaped filler (B-2) with an average particle diameter of 0.5 to 6.0 µm, and an irregularly shaped fine particulate filler (B-3) with an average particle diameter of less than 0.05 µm.

Concerning the content of each component in the dental abutment construction material, it is preferable to comprise 15 parts or less by mass of hydrophilic polymerizable monomer (A-1a), 85 to 100 parts by mass of hydrophobic polymerizable monomer (A-1b), and 0 to 15 parts by mass of polymerizable monomer (A-2) having an acidic group, more preferably 1 to 10 parts by mass of hydrophilic polymerizable monomer (A-1a), 90 to 99 parts by mass of hydrophobic polymerizable monomer (A-1b), and 0 to 9 parts by mass of polymerizable monomer (A-2) having an acidic group, even more preferably 2 to 5 parts by mass of hydrophilic polymerizable monomer (A-1a), 95 to 98 parts by mass of hydrophobic polymerizable monomer (A-1b), and 0 to 3 parts by mass of polymerizable monomer (A-2) having an acidic group in total 100 parts by mass of the polymerizable monomer (A) in the dental curable composition. It is also preferable to comprise 1.0 to 900 parts by mass of filler (B-1), 40 to 1,485 parts by mass of filler (B-2), and 0.1 to 150 parts by mass of filler (B-3), more preferably 7.5 to 500 parts by mass of filler (B-1), 74.85 to 949 parts by mass of filler (B-2), and 0.15 to 80 parts by mass of filler (B-3), even more preferably 20 to 150 parts by mass of filler (B-1), 138 to 445 parts by mass of filler (B-2), and 2 to 25 parts by mass of filler (B-3) with respect to total 100 parts by mass of polymerizable monomer (A). It is also preferable to comprise 0.001 to 20 parts by mass of polymerization initiator (C), and 0.001 to 40 parts by mass of polymerization accelerator (D), more preferably 0.05 to 10 parts by mass of polymerization initiator (C), and 0.05 to 20 parts by mass of polymerization accelerator (D) with respect to total 100 parts by mass of polymerizable monomer (A). The dental curable composition, when used as a dental abutment construction material, is not required to comprise a hydrophilic polymerizable monomer (A-1a). When used as a type of dental abutment construction material used with a pretreatment material, the dental curable composition is not required to comprise a polymerizable monomer (A-2) having an acidic group.

In any of the preferred embodiments as dental cements, self-adhesive dental cements, and dental abutment construction materials, the content of each component may be varied as appropriate based on the descriptions provided in this specification, and changes such as addition and deletion can be made for any of the components.

The present invention encompasses embodiments combining the foregoing features in various ways within the technical scope of the present invention, provided that the present invention can exhibit its effects.

### EXAMPLES

The following describes the present invention in greater detail through Examples. However, the present invention is in no way limited by the following Examples.

The components of the dental curable compositions of Examples and Comparative Examples are presented below, along with the abbreviations.
[Hydrophilic polymerizable monomer (A-1a)]
   #801: 1,2-bis(3-methacryloyloxy-2-hydroxypropoxy)ethane
   3G: triethylene glycol dimethacrylate
[Hydrophobic polymerizable monomer (A-1b)]
   Bis-GMA: 2,2-bis[4-(2-hydroxy-3-methacryloyloxypropoxy)phenyl]propane
   NPG: neopentyl glycol dimethacrylate
   D-2.6E: 2,2-bis(4-methacryloyloxypolyethoxyphenyl)propane (with an average of 2.6 moles of ethoxy group added)
[Polymerizable monomer (A-2) having an acidic group]
   MDP: 10-methacryloyloxydecyl dihydrogen phosphate
[Spherical filler (B-1)]
   Filler 1: The filler produced in the Production Example 1-1 below.
   Filler 2: The filler produced in the Production Example 1-2 below.
[Irregularly shaped filler (B-2)]
   Filler 3: The filler produced in the Production Example 2-1 below.
   Filler 4: The filler produced in the Production Example 2-2 below.
[Irregularly shaped fine particulate filler (B-3)]
   Filler 5: The filler produced in the Production Example 3-1 below.

### [Production Example 1-1]

### · Production of Filler 1

Sixty parts by mass of isopropanol was added to one-hundred parts by mass of silica particles (SNOWTEX^{®} OL manufactured by Nissan Chemical Industries, Ltd. under this trade name; colloidal silica, a water dispersion with a solid concentration of 20 mass%, average particle diameter: 50 nm, average uniformity: 0.8 or more), and these were mixed at room temperature (about 25°C) to obtain a dispersion. To this dispersion, 0.48 parts by mass of 3-methacryloyloxypropyltrimethoxysilane (KBM-503 manufactured by Shin-Etsu Chemical Co., Ltd. under this trade name) and 0.01 parts by mass of a polymerization inhibitor (2,6-di-t-butyl-4-methylphenol (BHT), manufactured by Kanto Kagaku) were added, and these were mixed at 40°C for 72 hours. Subsequently, 0.78 parts by mass of 1,1,1,3,3,3-hexamethyldisilazane (HMDS-1 manufactured by Shin-Etsu Chemical Co., Ltd. under this trade name) was added into this mixture, and the resultant mixture was left to stand at 40°C for 72 hours. During this process, the surface treatment of silica particles progressed, causing the silica particles to acquire hydrophobic properties, making the particles unstable in water and isopropanol, resulting in the formation of aggregates and precipitation. This was followed by adding 2.6 parts by mass of a 35% hydrochloric acid aqueous solution to the total amount of the mixture resulting from the surface treatment, inducing the precipitation of the silica particles. The resulting precipitates were filtered through filter paper (Quantitative Filter Paper No. 5A manufactured by Advantec Co., Ltd.). The resulting residue from the filtration process (solid component) was washed with purified water, and subjected to vacuum drying at 100°C to yield filler 1.

### [Production Example 1-2]

### · Production of Filler 2

Two-hundred parts by mass of isopropanol was added to one-hundred parts by mass of silica particles (SNOWTEX^{®} OS manufactured by Nissan Chemical Industries, Ltd. under this trade name; colloidal silica, a water dispersion with a solid concentration of 20 mass%, average particle diameter: 10 nm, average uniformity: 0.8 or more), and these were mixed at room temperature (about 25°C) to obtain a dispersion. To this dispersion, 2.4 parts by mass of 3-methacryloyloxypropyltrimethoxysilane (KBM-503 manufactured by Shin-Etsu Chemical Co., Ltd. under this trade name) and 0.01 parts by mass of a polymerization inhibitor (2,6-di-t-butyl-4-methylphenol (BHT), manufactured by Kanto Kagaku) were added, and these were mixed at 40°C for 72 hours. Subsequently, 3.9 parts by mass of 1,1,1,3,3,3-hexamethyldisilazane (HMDS-1 manufactured by Shin-Etsu Chemical Co., Ltd. under this trade name) was added into this mixture, and the resultant mixture was left to stand at 40°C for 72 hours. During this process, the surface treatment of silica particles progressed, causing the silica particles to acquire hydrophobic properties, making the particles unstable in water and isopropanol, resulting in the formation of aggregates and precipitation. This was followed by adding 7.8 parts by mass of a 35% hydrochloric acid aqueous solution to the total amount of the mixture resulting from the surface treatment, inducing the precipitation of the silica particles. The resulting precipitates were filtered through filter paper (Quantitative Filter Paper No. 5A manufactured by Advantec Co., Ltd.). The resulting residue from the filtration process (solid component) was washed with purified water, and subjected to vacuum drying at 100°C to yield filler 2.

### [Production Example 2-1]

### · Production of Filler 3

Eighty parts by mass of distilled water was added to one-hundred parts by mass of GM27884 UF2.0 grade (barium glass, manufactured by SCHOTT; average particle diameter: 2.0 µm), and these were mixed at room temperature (about 25°C) to obtain a dispersion. To this dispersion, 3.0 parts by mass of 3-methacryloyloxytrimethoxysilane (KBM-503 manufactured by Shin-Etsu Chemical Co., Ltd. under this trade name) and 200 parts by mass of a 0.3 mass% acetic acid aqueous solution were added in a three-neck flask, and the mixture was stirred at room temperature for 1 hour. After removing water by freeze drying, the resulting material was subjected to heat treatment at 90°C for 3 hours to yield filler 3.

### [Production Example 2-2]

### · Production of Filler 4

Two-hundred fifty parts by mass of isopropanol was added to one-hundred parts by mass of GM27884 UF2.0 grade (barium glass, manufactured by SCHOTT; average particle diameter: 2.0 µm), and these were mixed at room temperature (about 25°C) to obtain a dispersion. To this dispersion, 1.4 parts by mass of 8-methacryloyloxyoctyltrimethoxysilane (KBM-5803 manufactured by Shin-Etsu Chemical Co., Ltd. under this trade name), 5 parts by mass of a 0.3 mass% acetic acid aqueous solution, and 50 parts by mass of isopropanol were added in a three-neck flask, and the mixture was stirred at room temperature for 1 hour. After removing water by freeze drying, the resulting material was subjected to heat treatment at 90°C for 3 hours to yield filler 4.

### [Production Example 3-1]

### · Production of Filler 5

Fumed silica (Aerosil^{®} 130 manufactured by Nippon Aerosil Co., Ltd. under this trade name; average particle diameter: 0.016 µm) was subjected to surface treatment following an ordinary method using 3 parts by mass of 3-methacryloyloxypropyltrimethoxysilane with respect to 100 parts by mass of the fumed silica, yielding filler 5.
[Photopolymerization initiator (C-1)]
   CQ: camphorquinone
[Chemical polymerization initiator (C-2)]
   THP: 1,1,3,3-tetramethylbutyl hydroperoxide
[Photopolymerization accelerator (D-1)]
   PDE: ethyl 4-(N,N-dimethylamino)benzoate
[Chemical polymerization accelerator (D-2)]
   DMETU: 4,4-dimethylethylenethiourea
   VOAA: vanadyl(IV) acetylacetonate
[Polymerization inhibitor]
   BHT: 2,6-di-t-butyl-4-methylphenol

### Examples 1-1 to 1-24, 2-1 to 2-11, and Comparative Examples 1-1 to 1-9, 2-1 to 2-8 (Preparation of Dental Curable Composition)

The raw materials presented in Tables 1 to 5 were mixed and kneaded at ordinary temperature (25°C) in the dark to prepare paste-form dental curable compositions. The properties of these compositions were then examined following the methods of Test Examples 1 to 3 below. The results are presented in Tables 1 to 5.

### Examples 3-1 to 3-13, and Comparative Examples 3-1 to 3-8 (Preparation of Dental Curable Composition)

The raw materials presented in Tables 6 and 7 were mixed and kneaded at ordinary temperature (25°C) in the dark to prepare first and second agents of paste-form dental curable compositions, similarly to Examples 1-1 to 1-24, 2-1 to 2-11, and Comparative Examples 1-1 to 1-9, 2-1 to 2-8. Subsequently, the first and second agents, taken in equal quantities, were kneaded for 10 seconds, and their properties were assessed following the methods of Test Examples 1 to 3 below. The results are presented in Tables 6 and 7.

### Test Example 1: Consistency

The dental curable composition prepared in each Example and Comparative Example was left to stand for 1 day in a thermostatic chamber at 25°C (50% humidity), and 0.5 mL of the dental curable composition was weighed on a glass plate. Subsequently, a 40 g glass plate (5 cm × 5 cm) was placed on top, and, after 120 seconds, the long and short diameters of the sample were measured through the glass plate. The arithmetic average of these diameters were then calculated as consistency (mm) (n = 3).

When the diameter of the sample exceeded 50 mm and extended beyond the glass plate, it was recorded as having a consistency of 50 mm or more.

A higher consistency value indicates that the paste is softer, and consistency serves as an index commonly employed to evaluate the flowability of dental curable compositions.

A consistency of 20 mm to 40 mm is considered suitable for dental curable compositions of the present invention, with a range of 20 mm to 35 mm being more suitable, and a range of 20 mm to 30 mm being even more suitable.

### Test Example 2: Flexural Strength

The flexural strength was evaluated by a flexure test in compliance with ISO 4049: 2009, specifically as follows. The prepared paste (dental curable composition) was filled into a SUS die (2 mm in length × 25 mm in width × 2 mm in thickness), and the top and bottom surfaces (2 mm × 25 mm) of the paste were pressed with glass slides. Subsequently, the paste was cured by applying light to the top and bottom of the paste through the glass slides at five points on each side, 10 seconds per point, using a dental visible-light irradiator PenCure 2000 (manufactured by J. Morita Corp.).

The resulting cured product was measured for three-point flexural strength by conducting a flexure test at a span length of 20 mm and a crosshead speed of 1 mm/min, using a universal testing machine (Autograph AG-I 100kN, manufactured by Shimadzu Corporation), and the average value was calculated (n = 5).

Regarding the strength of a dental curable composition of the present invention, a strength of 100 MPa or more is considered suitable, with 110 MPa or more being more suitable, and 120 MPa or more being even more suitable.

### Test Example 3: Formability

A circle with a diameter of 3 mm was drawn on a 30 mm × 30 mm square glass plate, and 0.03 g of paste was weighed within this circle. Subsequently, the glass plate was horizontally placed in a thermostatic chamber set at 37°C, and the paste, left undisturbed for 60 seconds in this state, was visually examined for its shape. The paste shape (formability) was evaluated according to the evaluation criteria below (the lowest evaluation score was employed for n = 3).

For dental curable compositions of the present invention, those achieving a formability rating of 3 to 5 are considered suitable, and those with a formability rating of 4 or 5 are considered more suitable for the required level of formability as dental abutment construction materials.

### [Formability Evaluation Criteria]

5: The paste retains its shape observed at the time of weight measurement, without forming a hemispherical shape when viewed from the side
4: The paste forms a hemispherical shape when viewed from the side, but partially retains its shape observed at the time of weight measurement
3: The paste forms a hemispherical shape when viewed from the side, but the paste diameter, as viewed from above, remains unchanged from that at the time of weight measurement
2: The paste forms a hemispherical shape when viewed from the side, and the paste diameter, as viewed from above, is larger than that at the time of weight measurement
1: The paste fails to maintain its height observed at the time of weight measurement, resulting in a collapse.

Utilizing a paste with a formability rating of 4 or 5 enables the retention of the paste shape when the shaped paste is left to stand after building up and shaping the paste into the shape of an abutment tooth in abutment construction. Such pastes are particularly suited for use as dental abutment construction materials.

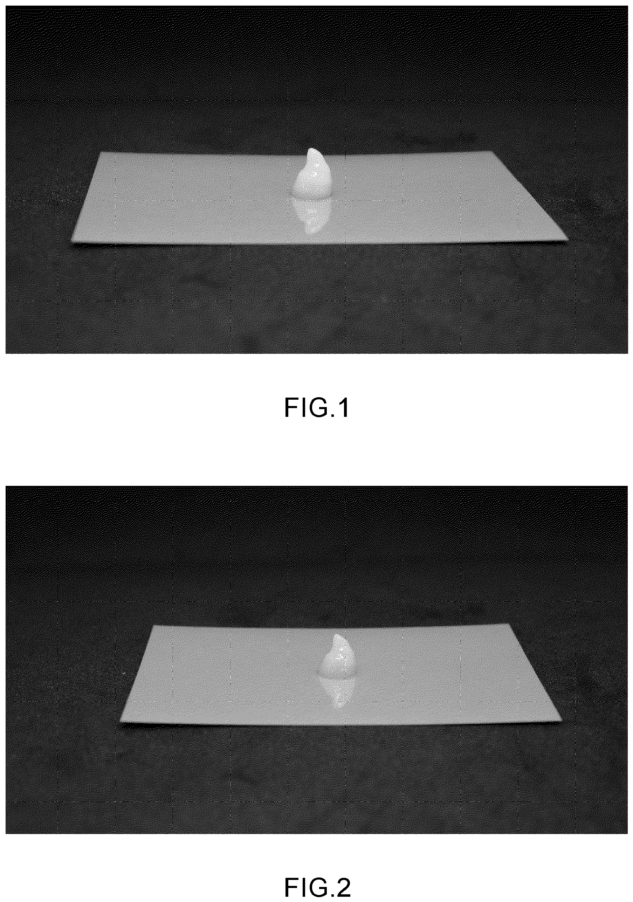

As an illustration of a paste of a dental curable composition of the present invention with a formability rating of 5, FIG. 1 shows a paste at the time of weight measurement, and FIG. 2 shows a paste after an elapsed time period of 10 minutes at 37°C (after a period of standing) from weight measurement.

As shown in FIG. 2, the paste is capable of maintaining its shape even after 10 minutes, eliminating concerns about the paste losing its form due to running or other factors during abutment construction in dental treatments.

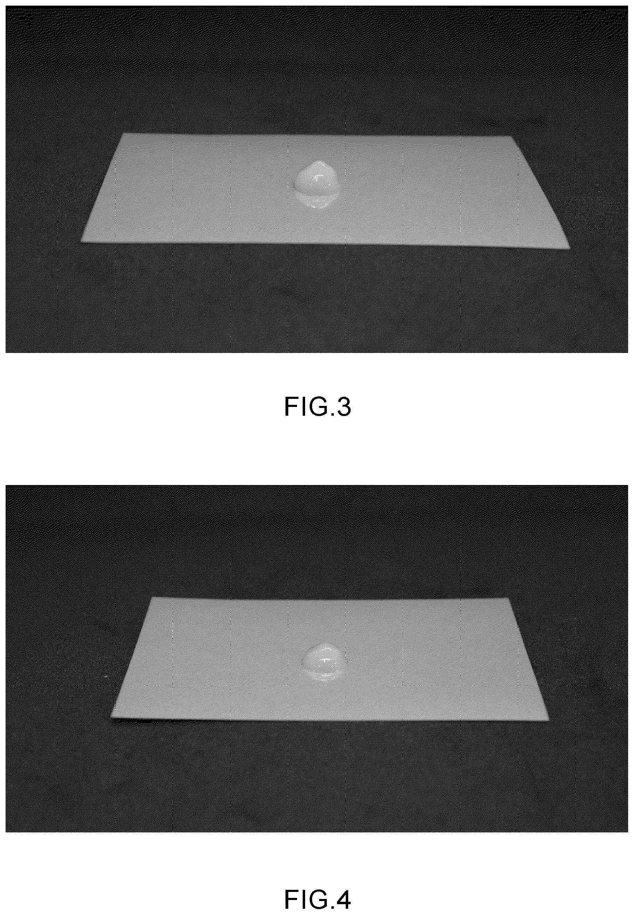

In contrast, as an illustration of a paste of a dental curable composition with a formability rating of 1, FIG. 3 shows a paste at the time of weight measurement (at the start of evaluation), and FIG. 4 shows a paste after an elapsed time period of 10 minutes at 37°C (after a period of standing) from the start of evaluation. As depicted in FIG. 3, the paste collapsed and lost its shape right after weight measurement, posing a possibility of being unable to retain the shape built during abutment construction.

Moreover, as depicted in FIG. 4, the paste underwent a change in shape after a predetermined time period, indicating that the paste is unable to retain the formed shape after it is built up in the construction of abutments in dental treatments.

**[Table 1]**

| Components (parts by mass with respect to 100 parts by mass of polymerizable monomer (A)) | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 | Ex. 1-11 | Ex. 1-12 |
|---|---|---|---|---|---|---|---|---|---|---|---|---|---|
| Hydrophilic polymerizable monomer (A-1a) | #801 | - | 5 | 9 | 13 | - | - | - | - | - | - | - | - |
| | 3G | - | - | - | - | 13 | - | - | - | - | - | - | - |
| Hydrophobic polymerizable monomer (A-1b) | Bis-GMA | 60 | 60 | 60 | 60 | 60 | 60 | 60 | 60 | 60 | 60 | 60 | 60 |
| | NPG | 40 | 35 | 31 | 27 | 27 | 40 | 40 | 40 | 40 | 40 | 40 | 25 |
| Polymerizable monomer having acidic group (A-2) | MDP | - | - | - | - | - | - | - | - | - | - | - | 15 |
| Spherical filler (B-1) | Filler 1 | 40 | 40 | 40 | 40 | 40 | 20 | - | 20 | 30 | 50 | 60 | 40 |
| | Filler 2 | - | - | - | - | - | 20 | 40 | - | - | - | - | - |
| Irregularly shaped filler (B-2) | Filler 3 | 140 | 140 | 140 | 140 | 140 | 140 | 140 | 160 | 150 | 130 | 120 | 140 |
| Fine particulate filler (B-3) | Filler 5 | - | - | - | - | - | - | - | - | - | - | - | - |
| Photopolymerization initiator (C-1) | CQ | 0.1 | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.1 | 0.1 | 0.1 | 0.1 | 0.1 | 0.1 | 0.1 | 0.1 | 0.1 | 0.1 | 0.1 | 0.1 |
| Polymerization inhibitor | BHT | 0.1 | 0.1 | 0.1 | 0.1 | 0.1 | 0.1 | 0.1 | 0.1 | 0.1 | 0.1 | 0.1 | 0.1 |
| Consistency mm | | 26 | 25 | 24 | 22 | 24 | 23 | 21 | 30 | 28 | 25 | 24 | 21 |
| Flexural strength MPa | | 116.4 | 117.6 | 117.6 | 121.4 | 123.6 | 117.6 | 120.6 | 125.4 | 126.0 | 116.4 | 111.6 | 110.2 |
| Formability | | 3 | 3 | 3 | 3 | 3 | 3 | 3 | 3 | 3 | 3 | 3 | 3 |

**[Table 2]**

| Components (parts by mass with respect to 100 parts by mass of polymerizable monomer (A)) | | Ex. 1-13 | Ex. 1-14 | Ex. 1-15 | Ex. 1-16 | Ex. 1-17 | Ex. 1-18 | Ex. 1-19 | Ex. 1-20 | Ex. 1-21 | Ex. 1-22 | Ex. 1-23 | Ex. 1-24 |
|---|---|---|---|---|---|---|---|---|---|---|---|---|---|
| Hydrophilic polymerizable monomer (A-1a) | #801 | - | 5 | 9 | 13 | - | - | - | - | - | - | - | - |
| | 3G | - | - | - | - | 13 | - | - | - | - | - | - | - |
| Hydrophobic polymerizable monomer (A-1b) | Bis-GMA | 60 | 60 | 60 | 60 | 60 | 60 | 60 | 60 | 60 | 60 | 60 | 60 |
| | NPG | 40 | 35 | 31 | 27 | 27 | 40 | 40 | 40 | 40 | 40 | 40 | 25 |
| Polymerizable monomer having acidic group (A-2) | MDP | - | - | - | - | - | - | - | - | - | - | - | 15 |
| Spherical filler (B-1) | Filler 1 | 40 | 40 | 40 | 40 | 40 | 20 | - | 20 | 30 | 50 | 60 | 40 |
| | Filler 2 | - | - | - | - | - | 20 | 40 | - | - | - | - | - |
| Irregularly shaped filler (B-2) | Filler 3 | 138 | 133 | 122 | 138 | 138 | 138 | 138 | 158 | 148 | 128 | 118 | 138 |
| Fine particulate filler (B-3) | Filler 5 | 2 | 9 | 18 | 2 | 2 | 2 | 2 | 2 | 2 | 2 | 2 | 2 |
| Photopolymerization initiator (C-1) | CQ | 0.1 | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.1 | 0.1 | 0.1 | 0.1 | 0.1 | 0.1 | 0.1 | 0.1 | 0.1 | 0.1 | 0.1 | 0.1 |
| Polymerization inhibitor | BHT | 0.1 | 0.1 | 0.1 | 0.1 | 0.1 | 0.1 | 0.1 | 0.1 | 0.1 | 0.1 | 0.1 | 0.1 |
| Consistency mm | | 25 | 22 | 21 | 21 | 23 | 22 | 20 | 29 | 27 | 24 | 22 | 20 |
| Flexural strength MPa | | 117.3 | 118.6 | 118.4 | 122.3 | 123.7 | 118.6 | 121.0 | 126.2 | 126.8 | 116.6 | 111.7 | 111.0 |
| Formability | | 4 | 5 | 5 | 4 | 4 | 4 | 4 | 4 | 4 | 4 | 4 | 4 |

**[Table 3]**

| Components (parts by mass with respect to 100 parts by mass of polymerizable monomer (A)) | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 | Com. Ex. 1-8 | Com. Ex. 1-9 |
|---|---|---|---|---|---|---|---|---|---|---|
| Hydrophilic polymerizable monomer (A-1a) | #801 | 20 | 20 | 40 | - | - | - | - | - | - |
| | 3G | - | - | - | 40 | - | - | - | - | - |
| Hydrophobic polymerizable monomer (A-1b) | Bis-GMA | 60 | 60 | 60 | 60 | 60 | 60 | 60 | 60 | 60 |
| | NPG | 20 | 20 | - | - | 40 | 40 | 40 | 40 | 40 |
| Spherical filler (B-1) | Filler 1 | 40 | 40 | 40 | 40 | 20 | - | - | 110 | 180 |
| Irregularly shaped filler (B-2) | Filler 3 | 140 | 139 | 140 | 140 | 140 | 180 | 160 | 70 | - |
| Fine particulate filler (B-3) | Filler 5 | - | 1 | - | - | 20 | - | 20 | - | - |
| Photopolymerization initiator (C-1) | CQ | 0.1 | 0.1 | 0.1 | 0.1 | 0.1 | 0.1 | 0.1 | 0.1 | 0.1 |
| Photopolymerization accelerator (D-1) | PDE | 0.1 | 0.1 | 0.1 | 0.1 | 0.1 | 0.1 | 0.1 | 0.1 | 0.1 |
| Polymerization inhibitor | BHT | 0.1 | 0.1 | 0.1 | 0.1 | 0.1 | 0.1 | 0.1 | 0.1 | 0.1 |
| Consistency mm | | 17 | 15 | 15 | 16 | 15 | 36 | 15 | 18 | 20 |
| Flexural strength MPa | | 117.2 | 118.2 | 118.1 | 121.2 | 105.3 | 116.6 | 109.5 | 101.3 | 85.2 |
| Formability | | 3 | 4 | 3 | 4 | 5 | 1 | 3 | 2 | 2 |

**[Table 4]**

| Components (parts by mass with respect to 100 parts by mass of polymerizable monomer (A)) | | Ex. 2-1 | Ex. 2-2 | Ex. 2-3 | Ex. 2-4 | Ex. 2-5 | Ex. 2-6 | Ex. 2-7 | Ex. 2-8 | Ex. 2-9 | Ex. 2-10 | Ex. 2-11 |
|---|---|---|---|---|---|---|---|---|---|---|---|---|
| Hydrophilic polymerizable monomer (A-1a) | #801 | - | 5 | 9 | 13 | - | - | - | - | - | - | - |
| Hydrophobic polymerizable monomer (A-1b) | Bis-GMA | 60 | 60 | 60 | 60 | 60 | 60 | 60 | 60 | 60 | 60 | 60 |
| | NPG | 40 | 35 | 31 | 27 | 40 | 40 | 40 | 40 | 40 | 40 | 25 |
| Polymerizable monomer having acidic group (A-2) | MDP | - | - | - | - | - | - | - | - | - | - | 15 |
| Spherical filler (B-1) | Filler 1 | 40 | 40 | 40 | 40 | 20 | - | 20 | 30 | 50 | 60 | 40 |
| | Filler 2 | - | - | - | - | 20 | 40 | - | - | - | - | - |
| Irregularly shaped filler (B-2) | Filler 4 | 138 | 132 | 122 | 138 | 138 | 138 | 158 | 148 | 128 | 118 | 138 |
| Fine particulate filler (B-3) | Filler 5 | 2 | 8 | 18 | 2 | 2 | 2 | 2 | 2 | 2 | 2 | 2 |
| Photopolymerization initiator (C-1) | CQ | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.1 | 0.1 | 0.1 | 0.1 | 0.1 | 0.1 | 0.1 | 0.1 | 0.1 | 0.1 | 0.1 |
| Polymerization inhibitor | BHT | 0.1 | 0.1 | 0.1 | 0.1 | 0.1 | 0.1 | 0.1 | 0.1 | 0.1 | 0.1 | 0.1 |
| Consistency mm | | 26 | 24 | 23 | 23 | 23 | 22 | 30 | 29 | 26 | 23 | 22 |
| Flexural strength MPa | | 121.4 | 118.8 | 122.5 | 128.1 | 121.5 | 122.6 | 128.0 | 127.7 | 118.4 | 115.0 | 113.2 |
| Formability | | 4 | 5 | 5 | 4 | 4 | 4 | 4 | 4 | 4 | 4 | 4 |

**[Table 5]**

| Components (parts by mass with respect to 100 parts by mass of polymerizable monomer (A)) | | Com. Ex. 2-1 | Com. Ex. 2-2 | Com. Ex. 2-3 | Com. Ex. 2-4 | Com. Ex. 2-5 | Com. Ex. 2-6 | Com. Ex. 2-7 | Com. Ex. 2-8 |
|---|---|---|---|---|---|---|---|---|---|
| Hydrophilic polymerizable monomer (A-1a) | #801 | 20 | 20 | 40 | - | - | - | - | - |
| Hydrophobic polymerizable monomer (A-1b) | Bis-GMA | 60 | 60 | 60 | 60 | 60 | 60 | 60 | 60 |
| | NPG | 20 | 20 | - | 40 | 40 | 40 | 40 | 40 |
| Spherical filler (B-1) | Filler 1 | 40 | 40 | 40 | 20 | - | - | - | 110 |
| Irregularly shaped filler (B-2) | Filler 4 | 140 | 139 | 140 | 140 | 180 | 160 | 150 | 70 |
| Fine particulate filler (B-3) | Filler 5 | - | 1 | - | 20 | - | 20 | 30 | - |
| Photopolymerization initiator (C-1) | CQ | 0.1 | 0.1 | 0.1 | 0.1 | 0.1 | 0.1 | 0.1 | 0.1 |
| Photopolymerization accelerator (D-1) | PDE | 0.1 | 0.1 | 0.1 | 0.1 | 0.1 | 0.1 | 0.1 | 0.1 |
| Polymerization inhibitor | BHT | 0.1 | 0.1 | 0.1 | 0.1 | 0.1 | 0.1 | 0.1 | 0.1 |
| Consistency mm | | 18 | 17 | 16 | 16 | 42 | 18 | 13 | 20 |
| Flexural strength MPa | | 114.8 | 114.9 | 116.9 | 119.9 | 115.3 | 114.2 | 101.6 | 88.9 |
| Formability | | 3 | 4 | 3 | 5 | 1 | 3 | 4 | 2 |

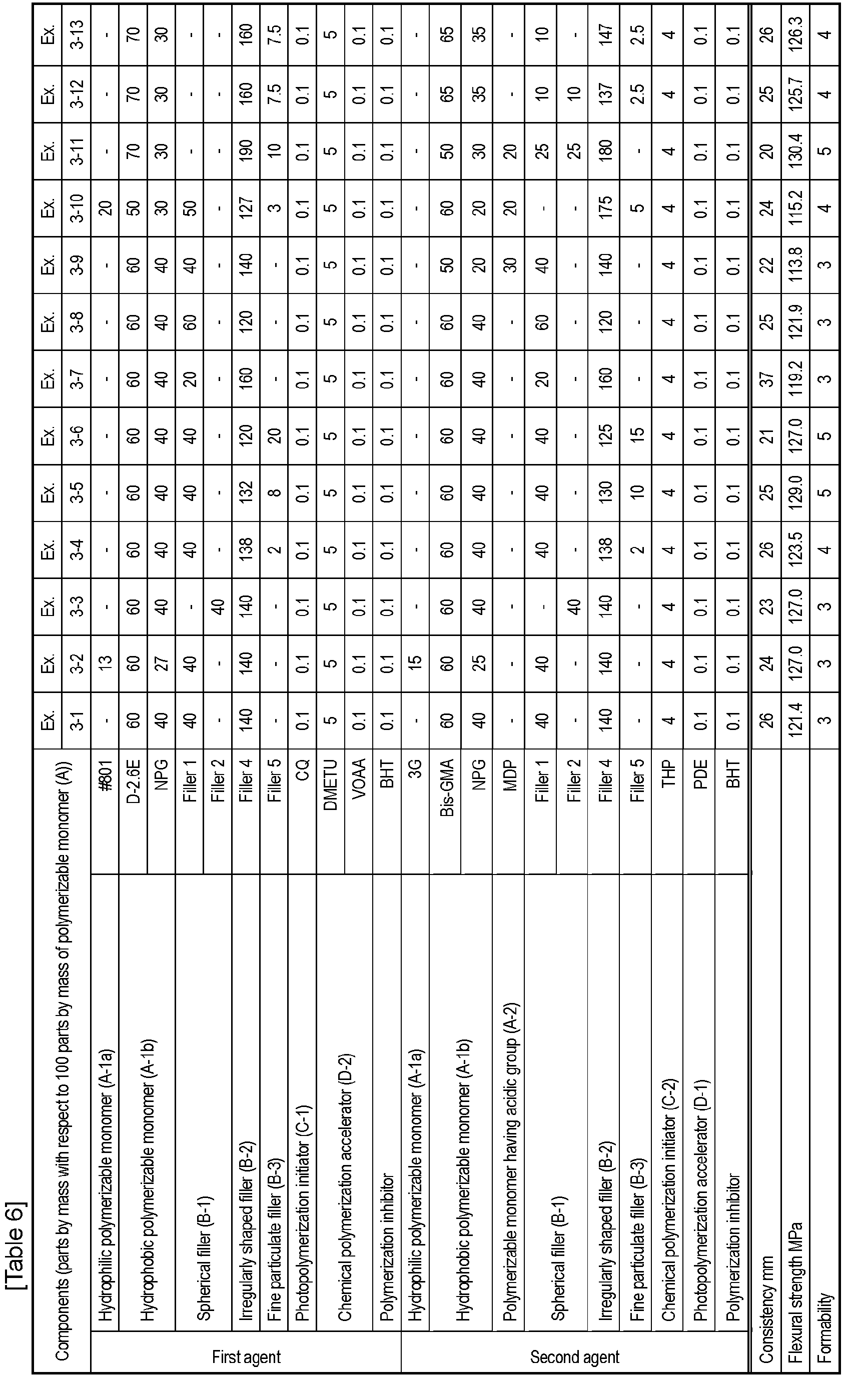

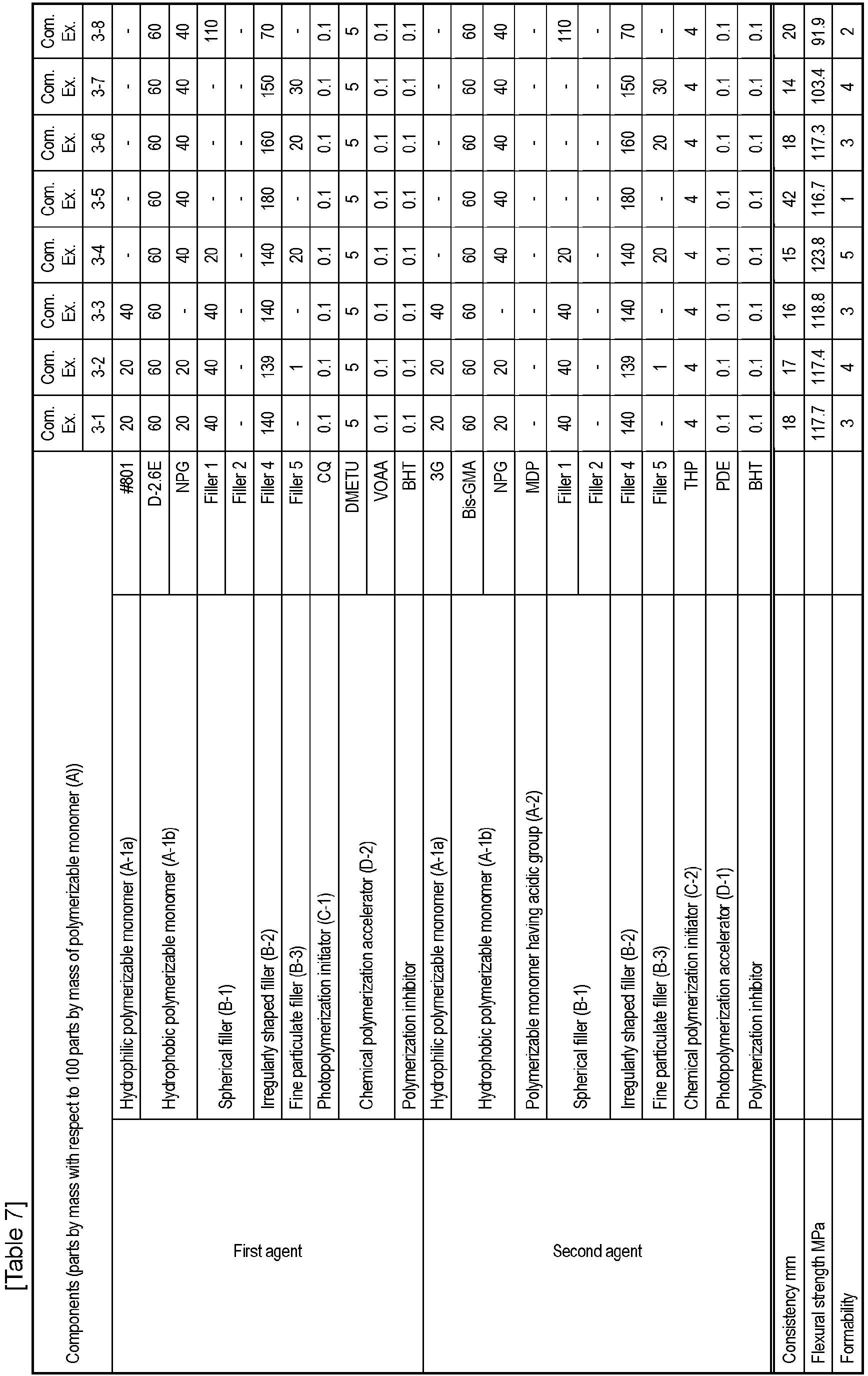

It can be seen from the results presented in the tables that the dental curable compositions of Examples exhibit outstanding mechanical strength, and, with the consistency falling in the range of 20 to 40 mm, demonstrate high paste flowability while possessing sufficient formability.

### INDUSTRIAL APPLICABILITY

A dental curable composition according to the present invention can be suitably used in applications such as dental cements, self-adhesive dental cements, and dental abutment construction materials in the field of dentistry.

## Claims

1. A dental curable composition comprising a polymerizable monomer (A), a filler (B), and a polymerization initiator (C), wherein:
the polymerizable monomer (A) comprises 0 parts or more by mass and less than 15 parts by mass of a hydrophilic polymerizable monomer (A-1a) having no acidic group, and 55 parts or more by mass of a hydrophobic polymerizable monomer (A-1b) having no acidic group in total 100 parts by mass of the polymerizable monomer (A), and the hydrophilic polymerizable monomer (A-1a) having no acidic group has a solubility of 4.5 g/L or more in water at 25°C,
the filler (B) comprises 1 to 60 parts by mass of a spherical filler (B-1) having an average particle diameter of 0.01 to 1 µm, 40 to 99 parts by mass of an irregularly shaped filler (B-2) having an average particle diameter of 0.5 to 6.0 µm, and 0 to 10 parts by mass of an irregularly shaped fine particulate filler (B-3) having an average particle diameter of less than 0.5 µm in total 100 parts by mass of the filler (B), and the spherical filler (B-1) is hydrophobized with a hydrophobizing agent (x-1), and
the polymerization initiator (C) comprises a photopolymerization initiator (C-1).

2. The dental curable composition according to claim 1, wherein the irregularly shaped fine particulate filler (B-3) has an average particle diameter of less than 0.05 µm.

3. The dental curable composition according to claim 1 or 2, wherein the filler (B) comprises 10 to 30 parts by mass of the spherical filler (B-1), 69 to 89 parts by mass of the irregularly shaped filler (B-2), and 1 to 5 parts by mass of the irregularly shaped fine particulate filler (B-3) in total 100 parts by mass of the filler (B).

4. The dental curable composition according to any one of claims 1 to 3, wherein the hydrophobizing agent (x-1) comprises at least one selected from the group consisting of a silane coupling agent, a titanate-based coupling agent, an aluminate-based coupling agent, and a zirco-aluminate-based coupling agent.

5. The dental curable composition according to any one of claims 1 to 4, wherein the irregularly shaped filler (B-2) is hydrophobized with a hydrophobizing agent (x-2).

6. The dental curable composition according to claim 5, wherein the hydrophobizing agent (x-2) is a silane coupling agent having a polymerizable group and represented by the following general formula (1),
Y-SiRₙX₍₃₋ₙ₎ (1),
wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, n represents an integer of 0, 1, or 2, and R may be the same or different from each other when R is plural, and X may be the same or different from each other when X is plural.

7. The dental curable composition according to claim 6, wherein Y is a group with a (meth)acryloyl group and an organic group bound to each other via a divalent group containing an oxygen atom, and the organic group bound to the (meth)acryloyl group via a divalent group containing an oxygen atom is an alkyl group having 5 to 11 carbon atoms.

8. The dental curable composition according to any one of claims 1 to 7, wherein the polymerizable monomer (A) further comprises a polymerizable monomer (A-2) having an acidic group.

9. The dental curable composition according to any one of claims 1 to 8, wherein the polymerization initiator (C) further comprises a chemical polymerization initiator (C-2).

10. The dental curable composition according to claim 9, which comprises a first agent and a second agent,
the first agent comprising the polymerizable monomer (A), the filler (B), and a chemical polymerization accelerator (D-2),
the second agent comprising the polymerizable monomer (A), the filler (B), and the chemical polymerization initiator (C-2).

11. The dental curable composition according to claim 10, wherein the chemical polymerization accelerator (D-2) comprises a thiourea compound and/or a salt of an aromatic sulfinic acid.

12. A dental abutment construction material comprising a dental curable composition of any one of claims 1 to 11.

13. A dental resin cement comprising a dental curable composition of any one of claims 1 to 11.
